# EUROPEAN PATENT SPECIFICATION

(11) **EP 2 167 084 B1**
(45) Date of publication and mention of the grant of the patent: **24.04.2013**
(21) Application number: 08760866.7
(22) Date of filing: 11.06.2008
(51) Int. Cl.: A61K 9/00, A61K 31/445, A61K 31/4545, A61K 31/495, A61K 31/506, A61K 39/35, A61K 39/36, A61P 37/08, A61K 9/19, A61K 9/20, A61K 39/00, A61K 45/06

(54) **AN ALLERGEN DOSAGE FORM COMPRISING AN ANTIHISTAMINE**
ALLERGEN-DOSIERFORM MIT EINEM ANTIHISTAMIN
FORME DE DOSAGE D'ALLERGÈNE CONTENANT UNE ANTIHISTAMINE

(30) Priority: 12.06.2007 EP 07011447; 12.06.2007 US 943373 P; 21.06.2007 DK 200700898
(43) Date of publication of application: 31.03.2010
(73) Proprietor: ALK-Abelló A/S, 2970 Hørsholm (DK)
(72) Inventor: JACOBI, Henrik Hugo, 2920 Charlottenlund (DK)
(74) Representative: Inspicos A/S
(86) International application number: PCT/EP2008/057317
(87) International publication number: WO 2008/152067

(56) References cited:
- WO-A-00/51568
- WO-A-02/078736
- WO-A-2005/077410
- WO-A-2006/008512
- US-A1- 2004 166 123
- PRADALIER A ET AL: "Sublingual-swallow immunotherapy (SLIT) with a standardized five-grass-pollen extract (drops and sublingual tablets) versus placebo in seasonal rhinitis" ALLERGY, MUNSKGAARD, COPENHAGEN, DK, vol. 54, no. 8, August 1999 (1999-08), pages 819-828, XP002403022 ISSN: 0105-4538
- KORSGREN MAGNUS ET AL: "Clinical efficacy and pharmacokinetic profiles of intranasal and oral cetirizine in a repeated allergen challenge model of allergic rhinitis." ANNALS OF ALLERGY, ASTHMA & IMMUNOLOGY : OFFICIAL PUBLICATION OF THE AMERICAN COLLEGE OF ALLERGY, ASTHMA, & IMMUNOLOGY APR 2007, vol. 98, no. 4, April 2007 (2007-04), pages 316-321, XP009092393 ISSN: 1081-1206
- PHAM-THI NHÂN ET AL: "Assessment of sublingual immunotherapy efficacy in children with house dust mite-induced allergic asthma optimally controlled by pharmacologic treatment and mite-avoidance measures." PEDIATRIC ALLERGY AND IMMUNOLOGY : OFFICIAL PUBLICATION OF THE EUROPEAN SOCIETY OF PEDIATRIC ALLERGY AND IMMUNOLOGY FEB 2007, vol. 18, no. 1, February 2007 (2007-02), pages 47-57, XP002459428 ISSN: 0905-6157

## Description

### Field of the Invention

The invention relates to an allergen containing pharmaceutical product and in particular to allergen dosage units comprising a low amount of an antihistamine and a method for preparing and using such dosage units.

### Background of the Invention

Allergy is a major health problem in countries where Western lifestyle is adapted. Furthermore, the prevalence of allergic disease is increasing in these countries. Although allergy in general may not be considered a life-threatening disease, asthma annually causes a significant number of deaths. An exceptional prevalence in about 30% of teenagers conveys a substantial loss in quality of life, working days and money, and warrants a classification among major health problems in the Western world.

Allergy is a complex disease. Many factors contribute to the sensitisation event. Among these is the susceptibility of the individual defined by an as yet insufficiently understood interplay between several genes. Another important factor is allergen exposure above certain thresholds. Several environmental factors may be important in the sensitisation process including pollution, childhood infections, parasite infections, intestinal microorganisms, etc. Once an individual is sensitised and the allergic immune response established, the presence of only minute amounts of allergen is efficiently translated into symptoms.

The natural course of allergic disease is usually accompanied by aggravation at two levels. First, there is a progression of symptoms and disease severity. For example, there is a progression from hay fever to asthma. Secondly, dissemination in offending allergens most often occurs resulting in allergic multi-reactivity. Chronic inflammation leads to a general weakening of the mucosal defense mechanisms resulting in unspecific irritation and eventually destruction of the mucosal tissue. Infants may become sensitised primarily to foods, i.e. milk, resulting in eczema or gastrointestinal disorders; however, most often they outgrow these symptoms spontaneously. These infants are at risk of developing inhalation allergy later in their lives.

The most important allergen sources are found among the most prevalent particles of a certain size in the air we breathe. These sources are remarkably universal and include grass pollens and house dust mite faecal particles, which together are responsible for approximately 50% of all allergies. Of global importance are also animal dander, i.e. cat and dog dander, other pollens, such as mugwort pollens, and micro-fungi, such as Alternaria. On a regional basis other pollens may dominate, such as birch pollen in Northern and Central Europe, ragweed in the Eastern and Central United States, and Japanese cedar pollen in Japan. Insects, i.e. bee and wasp venoms, and foods each account for approximately 2% of all allergies.

Allergy, i.e. type I hyper-sensitivity, is caused by an inappropriate immunological reaction to foreign non-pathogenic substances. Important clinical manifestations of allergy include asthma, hay fever, eczema, and gastro intestinal disorders. The allergic reaction is prompt and peaks within 20 minutes upon contact with the offending allergen. Furthermore, the allergic reaction is specific in the sense that a particular individual is sensitised to particular allergen(s), whereas the individual does not necessarily show an allergic reaction to other substances known to cause allergic disease. The allergic phenotype is characterized by a pronounced inflammation of the mucosa of the target organ and by the presence of allergen specific antibody of the IgE class in the circulation and on the surfaced of mast-cells and basophils.

An allergic attack is initiated by the reaction of the foreign allergen with allergen specific IgE antibodies, when the antibodies are bound to high affinity IgE specific receptors on the surface of mast-cells and basophils. The mast-cells and basophils contain preformed mediators, i.e. histamine, tryptase, and other substances, which are released upon cross-linking of two or more receptor-bound IgE antibodies. IgE antibodies are cross-linked by the simultaneous binding of one allergen molecule. The cross-linking of receptor bound IgE on the surface of mast-cells also leads to release of signalling molecules responsible for the attraction of eosinophils, allergen specific T-cells, and other types of cells to the site of the allergic response. These cells in interplay with allergen, IgE and effector cells, lead to a renewed flash of symptoms occurring 12-24 hours after allergen encounter (late phase reaction).

Allergy disease management comprises diagnosis and treatment including prophylactic treatments. Diagnosis of allergy is concerned with the demonstration of allergen specific IgE and identification of the allergen source. In many cases a careful anamnesis may be sufficient for the diagnosis of allergy and for the identification of the offending allergen source material. Most often, however, the diagnosis is supported by objective measures, such as skin prick test, blood test, or provocation test.

The therapeutic options fall in three major categories. The first opportunity is allergen avoidance or reduction of the exposure. Whereas allergen avoidance is obvious e.g. in the case of food allergens, it may be difficult or expensive, as for house dust mite allergens, or it may be impossible, as for pollen allergens. The second and most widely used therapeutic option is the prescription of classical symptomatic drugs like anti-histamines and steroids. Symptomatic drugs are safe and efficient; however, they do not alter the natural cause of the disease, and they do not control the disease dissemination. The third therapeutic alternative is specific allergy vaccination that in most cases reduces or alleviates the allergic symptoms caused by the allergen in question.

Conventional specific allergy vaccination is a causal treatment for allergic disease. It interferes with basic immunological mechanisms resulting in persistent improvement of the patients' immune status. Thus, the protective effect of specific allergy vaccination extends beyond the treatment period in contrast to symptomatic drug treatment. Some patients receiving the treatment are cured, and in addition, most patients experience a relief in disease severity and symptoms experienced, or at least an arrest in disease aggravation. Thus, specific allergy vaccination has preventive effects reducing the risk of hay fever developing into asthma, and reducing the risk of developing new sensitivities.

The immunological mechanism underlying successful allergy vaccination is not known in detail. A specific immune response, such as the production of antibodies against a particular pathogen, is known as an adaptive immune response. This response can be distinguished from the innate immune response, which is an unspecific reaction towards pathogens. An allergy vaccine is bound to address the adaptive immune response, which includes cells and molecules with antigen specificity, such as T-cells and the antibody producing B-cells. B-cells cannot mature into antibody producing cells without help from T-cells of the corresponding specificity. T-cells that participate in the stimulation of allergic immune responses are primarily of the Th2 type. Establishment of a new balance between Th1 and Th2 cells has been proposed to be beneficial and central to the immunological mechanism of specific allergy vaccination. Whether this is brought about by a reduction in Th2 cells, a shift from Th2 to Th1 cells, or an up-regulation of Th1 cells is controversial. Recently, regulatory T-cells have been proposed to be important for the mechanism of allergy vaccination. According to this model regulatory T-cells, i.e. Th3 or Tr1 cells, down-regulate both Th1 and Th2 cells of the corresponding antigen specificity. In spite of these ambiguities it is generally believed that an active vaccine must have the capacity to stimulate allergen specific T-cells, preferably TH1 cells.

Primarily for two reasons specific allergy vaccination in spite of its virtues, is not in widespread use. One reason is the inconveniences associated with the traditional vaccination program that comprises repeated vaccinations, such as injections over several months. The other reason is, more importantly, the risk of allergic side reactions. Ordinary vaccinations against infectious agents are efficiently performed using a single or a few high dose immunizations. This strategy, however, cannot be used for allergy vaccination since a pathological immune response is already ongoing.

Conventional specific allergy vaccination is therefore carried out using multiple subcutaneous immunizations applied over an extended time period. The course is divided in two phases, the up dosing and the maintenance phase. In the up dosing phase increasing doses are applied, typically over a 16-week period, starting with minute doses. When the recommended maintenance dose is reached, this dose is applied for the maintenance phase, typically with injections every six weeks. Following each injection the patient must remain under medical attendance for 30 minutes due to the risk of anaphylactic side reactions, which in principle although extremely rare could be life-threatening. In addition, the clinic should be equipped to support emergency treatment. There is no doubt that a vaccine based on a different route of administration would eliminate or reduce the risk for allergic side reactions inherent in the current subcutaneous based vaccine as well as would facilitate a more widespread use, possibly even enabling self vaccination at home.

Attempts to improve vaccines for specific allergy vaccination have been performed for over 30 years and include multifarious approaches. Several approaches have addressed the allergen itself through modification of the IgE reactivity. Others have addressed this route of administration.

Oromucosal immunotherapy can be regarded as a way of inducing tolerance and inducing mucosal vaccination. The mucosa of the mouth is rich in dendritic cells with a strong potential for antigen presentation. The dendritic cells are believed to process the allergens and then migrate to the local lymph nodes where they present allergen derived peptides to allergen specific T cells. During sublingual immunotherapy this dendritic cell - T cell interaction is believed to induce T cells with regulatory potential or to increase the ratio of allergen specific Th1 cells to allergen specific Th2 cells. A number of immunological parameters monitored during the allergy vaccination may be suitable markers for effects or efficacy of the treatment, alone or in combination respectively. These include systemic and mucosal antibody responses e.g. specific IgA, IgG and IgE antibodies; cytokine levels e.g. INFgamma, IL-2, IL-4, IL-5, IL-10, IL-12 and TNF alpha in blood or mucosal secretions; activation, chemotaxis, proliferation, signaling, cytokine production and other responses of regulatory T-cells, Th1 cells, TH2 cells, CD8 cells, other T cell subsets or B-cells or NK cells, and cell surface marker expression such as CD (cluster of differentiation) markers e.g. CD4, CD8, CD23, CD25, CD62L, CLA, beta7, CCR9, CD69, CD45RO, CCR3, CXCR5, effector cell function such as total histamine content of basophils; eosinophil, basophil, lymphocyte, monocyte numbers in blood, tissue and secretions; eosinophil, basophil, lymphocyte, monocyte mediator release, cytokine production, activation, chemotaxis, proliferation, signalling and other responses.

The immune system is accessible through the oral cavity and oromucosal, e.g. sublingual administration of allergens is a known route of administration.

Conventionally, allergy vaccine using the oromucosal route consists of the periodic dosing of a solution of the allergen at intervals spaced apart by at least one day. In comparison, the therapeutic (accumulated) maintenance doses when administered via the oral mucosa exceed the maintenance of the comparable subcutaneous dose by a factor of 5-500.

Fast dispersing solid dosage forms, which readily release the active ingredient in the oral cavity are known in the art, see for example US application No. 2004/0166123 describing a fast dissolving allergen dosage form suitable for oromucosal administration.

A problem associated with oromucosal administration of allergens is that some allergic patients experience local allergic reactions, primarily pruritus of the mount, irritation of the throat, mouth oedema and eye pruritis following administration of allergen via the oral mucosa. Most of these reactions are mild or moderate in intensity, have an onset immediately after oromucosal administration of allergen and last from minutes to hours after intake. These side effects disappears in many patients during the first days or weeks of specific allergen immunotherapy. However, some patients experience these side effects for longer periods, i.e. more than 150 days.

According to the present invention it has been found that allergen directly absorbed via the oral mucosa together with a low dose of an antihistamine having only a local effect in the mouth may relieve the oral itching experienced by patients during the first days or first weeks of treatment, or even longer.

By the use of antihistamines administered together with the allergen via the oral mucosa, it is possible to continue treatment of patients which would otherwise have ceased taking the allergy vaccine due to the itching in the oral cavity.

By the use of antihistamines administered together with the allergen via the oral mucosa it may also be possible to increase the amount of allergen in the dosage units, without the patients experiencing itching or a worsening of the itching in the oral mucosa.

US 2004/0166123 (paragraph 0244) describes fast dissolving solid dosage forms which may comprise an antihistamine such as bromapheniramine, cetirizine, fexofenadine, cyproheptadine, dexchlorpheniramine, hydroxizine, ketofene, mequitazine, oxotomide, mizolastine, ebastine, astemizole, carbinoxamide, alimemazine, buclizine, cyclizine, hydrochlorate, doxylamine, tritoqualine Many of the antihistamines mentioned in this document are antihistamines which need to be metabolized before they have antihistaminic effect. The reference does not suggest the use of a low dose of antihistamines that need not be metabolized to have antihistaminic effect in connection with oromucosal administration of allergens.

WO 02/078736 suggests antiallergic pharmaceutical compositions comprising at least two active ingredients selected from (i) an allergen, (ii) an antihistamine, and (iii) an inhibitor of histamine syntheses. The purpose of the combination of two active ingredients is to offer a new means of treating allergies that are both preventive and curative. The antihistamines mentioned in this document are: Bromopheniramine, cetirizine, fexofenadine, cyproheptadine, dexchlorpheniramine, hydroxizine, ketotifene, loratidine, mequitazine, oxotomide, mizolastine, ebastine, astemizole, carbinoxamide, alimemazine, buclizine, cyclizine, hydrochlorate and doxylamine. A range of between 1 to 2000 mg of antihistamine compound is suggested as the amount of antihistamine compound in the pharmaceutical compositions. It is readily apparent that the document does not suggest the specific use of antihistamines which do not require matabolization before they act as antihistamines. In addition, the document only discloses a broad range of the amount of antihistamine, which apparently applies to antihistamines in general.

According to the present invention, only a low amount of antihistamine is necessary to provide relief from the itching caused by administration of allergen via the oral mucosa.

WO 05/077410 suggest the use of an antihistamine, in this case cetirizine to treat and prevent itching in the mouth following sublinqual/ oral administration of a liquid formulation containing allergens. The liquid pharmaceutical composition was formulated to contain 200 mg/ml cetirizine, so that the patient is given 10 mg of cetirizine with each 50 µL dose of liquid allergen composition. It is mentioned that the maintenance dose is 150 µL of the allergen composition comprising 67 mg/mL of cetirizine, i.e. about 10 mg cetirizine in 150 µL of the allergen composition containing the maintenance dose. 10 mg cetirizine is the recommended oral daily dose administered to achieve systemic effect.

The pharmaceutical products according to the present invention differs from the product described in WO 05/077410 in that the pharmaceutical product of the invention comprises a lower amount of antihistamine than described in WO 05/077410.

### Summary of the Invention

The present invention relates to a pharmaceutical product comprising one or a plurality of dosage unit(s) suitable for oromucosal administration comprising
(a) an allergen,
(b) an antihistamine and
(c) pharmaceutically acceptable excipient(s)
as defined in the claims.

The present invention also provides methods for producing the pharmaceutical product for use in methods for treating allergy by oromucosal administration of the dosage unit(s) of the pharmaceutical products.

### Detailed Description of the Invention

Adverse events or side effects are known to exist also in connection with allergy treatment. In particular treatment aiming at modulating an ongoing response in a sensitised individual may pose a risk for inducing side effects upon administration of allergen. Normally side effects seen in connection with oromucosal treatment are reported in the eye, nose, mouth, and the upper and lower airways. Most common are itching phenomena.

The present invention is based on a number of surprising findings, none of which could with a reasonable expectation of success be predicted a priori.

First, the invention is based on the finding that the itching in the oral mucosa caused by oromucosal administration of allergen to a mammal sensitized to the allergen, may be relieved by oromucosal administration of an antihistamine which do not require metabolization to be active as antihistamine. Further, it has been found that a lower amount of antihistamine is required to relieve the itching in the oral mucosa, than the dose needed to achieve systemic effect via the oral route.

A number of antihistamines are known in the art and a number of these antihistamines (e.g. ebastine, loratidin) need to be metabolized in the patient to have effect as antihistamines.

It has been found that compounds that do not require metabolization in humans to be active as antihistamines, such as cetirizin, desloratidin, fexofenadin, levocitirizin and mizolastin, are particularly useful according to the invention.

The antihistamines useful according to the invention are typically H1, and/or H2 antagonists or inverse agonists.

According to this disclosure, one dose of allergen is administered together with an amount of antihistamine which is up to 50 % w/w of the recommended oral daily dose to a human above 12 years of age.

The term "oral" refers to the administration of a drug by the oral route, including the swallowing of the drug followed by absorption of the drug via the gastrointestinal tract to achieve a systemic effect.

The term "plurality" means two or more.

As used herein the expression "up to X" (where X is the amount antihistamine in %w/w or in mg) means "up to and including X".

Where the amount of antihistamine is defined herein as a percentage followed by w/w and the expression "of the oral daily dose", the expression "of the oral daily dose" means "of the oral daily dose of the active ingredient, i.e. the antihistamine". ,

The dosage unit may be a solid dosage unit, such as a tablet, a lozenge, a capsule and/or a caplet.

The dosage unit may also be a liquid dosage unit, i.e. a volume of a liquid formulation containing one dose of allergen and an amount of antihistamine according to the invention. According to this embodiment, the pharmaceutical product of the invention may be a container, such as a flask, vial or ampoule comprising one or more liquid dosage units each comprising one dose of allergen and an amount of antihistamine according to the invention. In one embodiment, the flask, vial or ampoule contains a liquid formulation with one dose of allergen and an amount of antihistamine according to the invention.

The term "one dose of allergen" means any amount of allergen administered at a specific point in time, i.e. administered in a solid dosage unit or in a liquid dosage unit (e.g. a specific volume or number of drops) at a specific point in time.

Suitably, "one dose of allergen" is an effective dose of an allergen for desensitization and shall mean a dose which when taken once or repeatedly in a monodose or in incremental doses results in, for example, an adaptive immune response and thus serves as means to desensitize allergic patients.

An effective dose is also a dose which in some patients causes the above mentioned adverse effects.

In a preferred embodiment "one dose of allergen" is the daily dose of allergen. Suitable the daily dose of allergen is an effective dose of an allergen as described above.

The recommended oral daily dose of antihistamines in a human over 12 years of age depends on the particular antihistamine in question.

As used herein, the recommended daily oral dose in a human above 12 years of age is 10 mg/day for cetirizine, 5 mg/day for desloratidine, 120 mg/day for fexofenadine, 5 mg/day for levotirizine and 10 mg/day for mizolastine.

The oral daily doses for other antihistamines may be obtained from the health authorities' published official documents describing the recommended oral daily dose for a human above 12 years of age.

Preferred one dosage unit comprises one dose of allergen and up to 50 % w/w of said oral daily dose of antihistamine, more preferred up to 45 % w/w of said oral daily dose of antihistamine, more preferred up to 40 % w/w of said oral daily dose of antihistamine, more preferred up to 35 % w/w of said oral daily dose of antihistamine, more preferred up to 30 % w/w of said oral daily dose of antihistamine, more preferred up to 25 % w/w of said oral daily dose of antihistamine, more preferred up to 20 % w/w of said oral daily dose of antihistamine, more preferred up to 15 % w/w of said oral daily dose of antihistamine, more preferred up to 10% w/w of said oral daily dose of antihistamine, or more preferred between 1 % and 5 % w/w of said oral daily dose of antihistamine.

In further embodiments one dosage unit suitably comprises one dose of allergen and 1-50 % w/w of said oral daily dose of antihistamine, preferably 5-50 % w/w of said oral daily dose of antihistamine, more preferred 10-50 % w/w of said oral daily dose of antihistamine, more preferred 15-50 % w/w of said oral daily dose of antihistamine, more preferred 20-50 % w/w of said oral daily dose of antihistamine, more preferred 25-50 % w/w of said oral daily dose of antihistamine, more preferred 30-50 % w/w of said oral daily dose of antihistamine or more preferred 40-50 % w/w of said oral daily dose of antihistamine.

In further embodiments one dosage unit suitably comprises one dose of allergen and 1-40 % w/w of said oral daily dose of antihistamine, preferably 5-40 % w/w of said oral daily dose of antihistamine, more preferred 10-40 % w/w of said oral daily dose of antihistamine, more preferred 15-40 % w/w of said oral daily dose of antihistamine, more preferred 20-40 % w/w of said oral daily dose of antihistamine, more preferred 25-40 % w/w of said oral daily dose of antihistamine, or more preferred 30-40 % w/w of said oral daily dose of antihistamine.

In further embodiments one dosage unit suitably comprises one dose of allergen and 1-30 % w/w of said oral daily dose of antihistamine, preferably 5-30 % w/w of said oral daily dose of antihistamine, more preferred 10-30 % w/w of said oral daily dose of antihistamine, more preferred 15-30 % w/w of said oral daily dose of antihistamine, more preferred 20-30 % w/w of said oral daily dose of antihistamine, or more preferred 25-30 % w/w of said oral daily dose of antihistamine.

In further embodiments one dosage unit suitably comprises one dose of allergen and 1-25 % w/w of said oral daily dose of antihistamine, preferably 5-25 % w/w of said oral daily dose of antihistamine, more preferred 10-25 % w/w of said oral daily dose of antihistamine, more preferred 15-25 % w/w of said oral daily dose of antihistamine, or more preferred 20-25 % w/w of said oral daily dose of antihistamine.

In further embodiments one dosage unit suitably comprises one dose of allergen and 1-25 % w/w of said oral daily dose of antihistamine, preferably 5-25 % w/w of said oral daily dose of antihistamine, more preferred 10-25 % w/w of said oral daily dose of antihistamine, more preferred 15-25 % w/w of said oral daily dose of antihistamine, or more preferred 20-25 % w/w of said oral daily dose of antihistamine.

In further embodiments one dosage unit suitably comprises one dose of allergen and 1-20 % w/w of said oral daily dose of antihistamine, preferably 5-20 % w/w of said oral daily dose of antihistamine, more preferred 10-20 % w/w of said oral daily dose of antihistamine, or more preferred 15-20 % w/w of said oral daily dose of antihistamine.

In further embodiments one dosage unit suitably comprises one dose of allergen and 1-15 % w/w of said oral daily dose of antihistamine, preferably 5-15 % w/w of said oral daily dose of antihistamine, or more preferred 10-15 % w/w of said oral daily dose of antihistamine.

In further embodiments one dosage unit suitably comprises one dose of allergen and 1-10 % w/w of said oral daily dose of antihistamine, preferably 5-10 % w/w of said oral daily dose of antihistamine or 1 - 5 % w/w of said oral daily dose of antihistamine.

According to one embodiment of the invention, one dosage unit comprises one dose of allergen and up to 5 mg cetirizine, more preferred up to 2.5 mg cetirizine, more preferred up to 2 mg cetirizine, more preferred up to 1 mg cetirizine, more preferred up to 0.5 mg cetirizine, or most preferred between 0.1 mg cetirizine and 0.5 mg cetirizine.

According to a further embodiment of the invention, one dosage unit comprises one dose of allergen and 0.1 - 5 mg cetirizine, more preferred 0.5 - 5 mg cetirizine, more preferred 1 - 5 mg cetirizine, more preferred 2 - 5 mg cetirizine, and more preferred 2.5 - 5 mg cetirizine, more preferred 3 - 5 mg cetirizine, or more preferred 4 - 5 mg cetirizine.

In a further embodiment of the invention, one dosage unit comprises one dose of allergen and 0.1 - 4 mg cetirizine, more preferred 0.5 - 4 mg cetirizine, more preferred 1 - 4 mg cetirizine, more preferred 2 - 4 mg cetirizine, and more preferred 2.5 - 4 mg cetirizine, or more preferred 3 - 4 mg cetirizine.

In a further embodiment of the invention, one dosage unit comprises one dose of allergen and 0.1 - 3 mg cetirizine, more preferred 0.5 - 3 mg cetirizine, more preferred 1 - 3 mg cetirizine, more preferred 2 - 3 mg cetirizine, or more preferred 2.5 - 3 mg cetirizine.

In a further embodiment of the invention, one dosage unit comprises one dose of allergen and 0.1 - 2.5 mg cetirizine, more preferred 0.5 - 2.5 mg cetirizine, more preferred 1 - 2.5 mg cetirizine, and more preferred 2 - 2.5 mg cetirizine.

In a further embodiment of the invention, one dosage unit comprises one dose of allergen and 0.1 - 2 mg cetirizine, more preferred 0.5 - 2 mg cetirizine, and more preferred 1 - 2 mg cetirizine.

In a further embodiment of the invention, one dosage unit comprises one dose of allergen and 0.1 - 1 mg cetirizine, and more preferred 0.5 - 1 mg cetirizine or between 0.1 mg cetirizine and 0.5 mg cetirizine

According to another embodiment of the invention, one dosage unit comprises one dose of allergen and up to 2.5 mg desloratidine, more preferred up to 1.75 mg desloratidine more preferred up to 1.25 mg desloratidine, more preferred up to 1 mg desloratidine more preferred up to 0.5 mg desloratidine, or most preferred between 0.05 mg desloratidine and 0.25 mg desloratidine.

According to a further embodiment of the invention, one dosage unit comprises one dose of allergen and 0.05 - 2.5 mg desloratidine, more preferred 0.25 - 2.5 mg desloratidine, more preferred 0.5 - 2.5 mg desloratidine, more preferred 1 - 2.5 mg desloratidine, more preferred 1.5 - 2.5 mg desloratodine and more preferred 2 - 2.5 mg desloratidine.

According to a further embodiment of the invention, one dosage unit comprises one dose of allergen and 0.05 - 2 mg desloratidine, more preferred 0.25 - 2 mg desloratidine, more preferred 0.5 - 2 mg desloratidine, more preferred 1 - 2 mg desloratidine, or more preferred 1.5 - 2 mg desloratidine.

According to a further embodiment of the invention, one dosage unit comprises one dose of allergen and 0.05 - 1.5 mg desloratidine, more preferred 0.25 - 1.5 mg desloratidine, and more preferred 0.5 - 1.5 mg desloratidine, or more preferred 1 -1.5 mg desloratidine.

According to a further embodiment of the invention, one dosage unit comprises one dose of allergen and 0.05 - 1 mg desloratidine, more preferred 0.25 - 1 mg desloratidine and more preferred 0.5 - 1 mg desloratidine.

According to a further embodiment of the invention, one dosage unit comprises one dose of allergen and 0.05 - 0,5 mg desloratidine, and more preferred 0.25 - 0,5 mg desloratidine or between 0.05 mg desloratidine and 0.25 mg desloratidine.

According to a further embodiment of the invention, one dosage unit comprises one dose of allergen and up to 60 mg fexofenadine, more preferred up to 30 mg fexofenadine, more preferred up to 12 mg fexofenadine, more preferred up to 2 mg fexofenadine, or most preferred between 1.2 mg fexofenadine and 6 mg fexofenadine, or below 1 mg fexofenadine.

According to still a further embodiment of the invention, one dosage unit comprises one dose of allergen and 1 - 60 mg fexofenadine, more preferred 2 - 60 mg fexofenadine, more preferred 10 - 60 mg fexofenadine, more preferred 20 - 60 mg fexofenadine, more preferred 30 - 60 mg fexofenadine, more preferred 40 - 60 mg fexofenadine, or more preferred 50 - 60 mg fexofenadine.

According to still a further embodiment of the invention, one dosage unit comprises one dose of allergen and 1 - 50 mg fexofenadine, more preferred 2 - 50 mg fexofenadine, more preferred 10 - 50 mg fexofenadine, more preferred 20 - 50 mg fexofenadine, more preferred 30 - 50 mg fexofenadine, or more preferred 40 - 50 mg fexofenadine.

According to still a further embodiment of the invention, one dosage unit comprises one dose of allergen and 1 - 40 mg fexofenadine, more preferred 2 - 40 mg fexofenadine, or more preferred 10 - 40 mg fexofenadine, more preferred 20 - 40 mg fexofenadine, or more preferred 30 - 40 mg fexofenadine.

According to still a further embodiment of the invention, one dosage unit comprises one dose of allergen and 1 - 30 mg fexofenadine, more preferred 2 - 30 mg fexofenadine, more preferred 10 - 30 mg fexofenadine or more preferred 20 - 30 mg fexofenadine.

According to still a further embodiment of the invention, one dosage unit comprises one dose of allergen and 1 - 20 mg fexofenadine, more preferred 2 - 20 mg fexofenadine, or more preferred 10 - 20 mg fexofenadine.

According to still a further embodiment of the invention, one dosage unit comprises one dose of allergen and 1 - 10 mg fexofenadine, more preferred 2 - 10 mg fexofenadine, and more preferred 1 - 2 mg fexofenadine.

According to a further embodiment of the invention, one dosage unit comprises one dose of allergen and up to 2.5 mg levocitirizine, more preferred up to 2 mg levozitirizine, more preferred up to 1.25 mg levozitirizine, more preferred up to 0.95 mg levozitirizine, more preferred up to 0.5 mg levozitirizine, or most preferred between 0.05 mg levozitirizine and 0.25 mg levozitirizine.

According to a further embodiment of the invention, one dosage unit comprises one dose of allergen and 0.05 - 2.5 mg levocitirizine, more preferred 0.25 - 2.5 mg levozitirizine, more preferred 0.5 - 2.5 mg levozitirizine, more preferred 1- 2.5 mg levozitirizine, more preferred 1.5 - 2.5 mg levozitirizine, and more preferred 2 - 2.5 mg levozitirizine.

According to a further embodiment of the invention, one dosage unit comprises one dose of allergen and 0.05 - 2 mg levocitirizine, more preferred 0.25 - 2 mg levozitirizine, more preferred 0.5 - 2 mg levozitirizine, more preferred 1- 2 mg levozitirizine, and more preferred 1.5 - 2 mg levozitirizine.

According to still an embodiment of the invention, one dosage unit comprises one dose of allergen and 0.05 - 1.5 mg levocitirizine, more preferred 0.25 - 1.5 mg levozitirizine, more preferred 0.5 - 1.5 mg levozitirizine, and more preferred 1 -1.5 mg levozitirizine.

According to still an embodiment of the invention, one dosage unit comprises one dose of allergen and 0.05 - 1 mg levocitirizine, more preferred 0.25 - 1 mg levozitirizine, and more preferred 0.5 - 1 mg levozitirizine.

According to still an embodiment of the invention, one dosage unit comprises one dose of allergen and 0.05 - 0.5 mg and more preferred 0.25 - 0.5 mg levozitirizine or 0.05 - 0.25 mg levocitirizine.

The invention also relates to an embodiment of the invention where one dosage unit comprises one dose of allergen and up to 5 mg mizolastine, more preferred up to 2.5 mg mizolastine, more preferred up to 2 mg mizolastine, more preferred up to 1 mg mizolastine, more preferred up to 0.95 mg mizolastine, or most preferred between 0.1 mg mizolastine and 0.5 mg mizolastine.

In still another embodiment where one dosage unit comprises one dose of allergen and 0.1 - 5 mg mizolastine, more preferred 0.5 - 5 mg mizolastine, more preferred 1 - 5 mg mizolastine, more preferred 2 - 5 mg mizolastine, more preferred 2.5 - 5 mg mizolastine, more preferred 3 - 5 mg mizolastine, or more preferred 4 - 5 mg mizolastine.

In still another embodiment where one dosage unit comprises one dose of allergen and 0.1 - 4 mg mizolastine, more preferred 0.5 - 4 mg mizolastine, more preferred 1 - 4 mg mizolastine, more preferred 2 - 4 mg mizolastine, more preferred 2.5 - 4 mg mizolastine, or more preferred 3 - 4 mg mizolastine.

In still another embodiment where one dosage unit comprises one dose of allergen and 0.1 - 3 mg mizolastine, more preferred 0.5 - 3 mg mizolastine, more preferred 1 - 3 mg mizolastine, more preferred 2 - 3 mg mizolastine, or more preferred 2.5 - 3 mg mizolastine.

In still another embodiment where one dosage unit comprises one dose of allergen and 0.1 - 2.5 mg mizolastine, more preferred 0.5 - 2.5 mg mizolastine, more preferred 1 - 2.5 mg mizolastine, and more preferred 2 - 2.5 mg mizolastine.

In still another embodiment where one dosage unit comprises one dose of allergen and 0.1 - 2 mg mizolastine, more preferred 0.5 - 2 mg mizolastine, or more preferred 1 - 2 mg.

In still another embodiment where one dosage unit comprises one dose of allergen and 0.1 - 1 mg mizolastine, or more preferred 0.5 - 1 mg mizolastine.

As indicated above the amount of antihistamine depends on the particular antihistamine in question.

Included in this invention are any pharmaceutically acceptable salts of the above mentioned antihistamines. The acids and bases from which these salts are prepared include but are not limited to the acids and bases listed herein. The acids include, but are not limited to, the following inorganic acids: hydrochloric acid, hydrobromic acid, hydroiodic acid, sulfuric acid and boric acid. The acids include, but are not limited to, the following organic acids: acetic acid, malonic acid, succinic acid, fumaric acid, tartaric acid, maleic acid, citric acid, methanesulfonic acid, benzoic acid, glycolic acid, lactic acid and mandelic acid. The bases include, but are not limited to ammonia, methylamine, ethylamine, propylamine, dimethylamine, diethylamine, trimethylamine, triethylamine, ethylenediamine, hydroxyethylamine, morpholine, piperazine and guanidine. This invention further provides for the hydrates and polymorphs of all of the compounds described herein.

The allergen may be a naturally occurring allergen. Examples of naturally occurring allergens include pollen allergens (tree, herb, weed, and grass pollen allergens), insect allergens (inhalant, saliva and venom allergens, e.g. mite allergens, cockroach and midges allergens, hymenoptera venom allergens), animal hair and dander allergens (from e.g. dog, cat, horse, rat, mouse, etc.), and food allergens. Important pollen allergens from trees, grasses and herbs are such originating from the taxonomic orders of Fagales, Oleales, Pinales and Platanaceae including for example birch (Betula), alder (Alnus), hazel (Corylus), hornbeam (Carpinus) and olive (Olea), cedar (Cryptomeria and Juniperus), Plane tree (Platanus), the order of Poales including for example grasses of the genera Lolium, Phleum, Poa, Cynodon, Dactylis, Holcus, Phalaris, Secale, and Sorghum, the orders of Asterales and Urticales including for example herbs of the genera Ambrosia, Artemisia, and Parietaria. Other important inhalation allergens are those from house dust mites of the genus Dermatophagoides and Euroglyphus, storage mite e.g. Blomia, Lepidoglyphys, Glycyphagus and Tyrophagus, those from cockroaches, midges and fleas e.g. Blatella, Periplaneta, Chironomus and Ctenocepphalides, and those from mammals such as cat (genus Felis), dog (genus Canis), cow (genus Bos) and horse (genus Equus), venom allergens including such originating from stinging or biting insects such as those from the taxonomic order of Hymenoptera including bees (superfamily Apidae), wasps (superfamily Vespidea), and ants (superfamily Formicoidae). Important inhalation allergens from fungi are, for example, those originating from the genera Alternaria and Cladosporium.

In a more preferred embodiment of the invention the allergen is Bet v 1, Aln g 1, Cor a 1 and Car b 1, Que a 1, Cry j 1, Cry j 2 , Cup a 1, Cup s 1, Jun a 1, Jun a 2, jun a 3, Ole e 1 , Lig v 1, Pla I 1, Pla a 2, Amb a 1, Amb a 2, Amb t 5, Art v 1, Art v 2 Par j 1 , Par j 2, Par j 3, Sal k 1, Ave e 1, Cyn d 1, Cyn d 7, Dac g 1, Fes p 1, Hol I 1, Lol p 1 and 5, Pha a 1, Pas n 1, Phl p 1, Phl p 5, Phl p 6, Poa p 1, Poa p 5, Sec c 1, Sec c 5, Sor h 1, Der f 1, Der f 2, Der p 1, Der p 2, , Der p 7, Der m 1, Eur m 2, Gly d 1, Lep d 2, Blo t 5, Blo t 1, Tyr p 2, Bla g 1, Bla g 2, Per a 1, Fel d 1, Can f 1, Can f 2 , Bos d 2, Equ c 1, Equ c 2, Equ c 3, Mus m 1, Rat n 1, Apis m 1, Api m 2 , Ves v 1, Ves v 2, Ves v 5, Dol m 1, Dol m 2, Dol m 5, Pol a 1, Pol a 2, Pol a 5, Sol i 1, Sol i 2, Sol i 3 and Sol i 4, Alt a 1, Cla h 1, Asp f 1, Bos d 4, Mal d 1, Gly m 1, Gly m 2, Gly m 3, Ara h 1, Ara h 2, Ara h 3, Ara h 4, Ara h 5 or shufflant hybrids from Molecular Breeding (Maxygen, Inc.) or any of these.

In a further preferred embodiment major allergens include grass group 1 allergen e.g. phl p 1, lol p 1 , sor h 1, dac g 1, cyn d 1, hol I 1, pha a 1, grass group 2/3 allergen e.g. phl p 2/3, lol p 2/3, grass group 5 allergen e.g. phl p 5, lol p 5, dac g 5, poa p 5, grass group 6 allergen e.g. phl p 6, poa p 6, tree pollen group 1 allergen e.g. bet v1, aln g 1, cor a 1, car b 1, mite group 1 allergen e.g. der p 1, der f 1, eur m 1, mite group 2 allergen e.g. der p 2, der f 2, eur m2, cat allergen e.g. fel d 1, cedar group 1 and group 2 allergen e.g. cry j 1, cry j 2, short or giant ragweed pollen allergen e.g. amb a 1, amb a 2, amb 1, amb t 2.

In the most preferred embodiment of the invention, the allergen is a grass pollen allergen or a mite allergen, such as a house dust mite allergen or a storage mite allergen, a ragweed allergen, a cedar pollen allergen, a cat allergen or a birch allergen.

For example the dosage unit comprises grass group 1, grass group 2/3, grass group 5 and grass group 6 allergens or mite group 1 and group 2 allergens from different grass and mite species respectively, weed antigens like short and giant ragweed allergens, different fungi allergens like alternaria and cladosporium, tree allergens like birch, hazel, hornbeam, oak and alder allergens, food allergens like peanut, soybean and milk allergens.

The allergen may be in the form of an extract, a purified allergen, a modified allergen, a recombinant allergen or a mutant of a recombinant allergen. An allergenic extract may naturally contain one or more isoforms of the same allergen, whereas a recombinant allergen typically only represents one isoform of an allergen. In a preferred embodiment the allergen is in the form of an extract. In another preferred embodiment the allergen is a recombinant allergen. In a further preferred embodiment the allergen is a naturally occurring low IgE-binding mutant or a recombinant low IgE-binding mutant.

In yet another embodiment of the invention the dosage unit(s) comprises at least two different types of allergens either originating from the same allergenic source or originating from different allergenic sources.

Allergens may be present in equi-molar amounts or the ratio of the allergens present may vary preferably up to 1:20.

In a further embodiment of the invention the low IgE binding allergen is an allergen according to WO 99/47680 or WO 02/40676 or PCT/DK03/00322 ("Allergen mutants").

It is known in the art that domestic and companion ship animal develop allergies toward numerous allergen sources including grass, house dust mites, and parasites. Hematophagous, i.e. bloodsucking insect infestation is known to lead to a hypersensitive response called flea allergic dermatitis (FAD). In a preferred embodiment of the current invention allergens for animal vaccines include allergens originating or transferred from parasites like ectoparasites (e.g. fleas, ticks, mosquitoes, flies), parasitic helminth venom (like heart worm e.g. Dirofilaria or onchocerciasis e.g. Onchocerca) and house dust mite. More preferred are saliva allergens from fleas like Ctenocephalides e.g. C. canis and C. felis, hard ticks likes Ixodes, Amblyomma, soft ticks like Omithodoros and from midges like Culicoides.

Guidance to the normally applied, acceptable tests measuring biopotency are found e.g. in Note for Guidance on Allergen Product; The European Agency for the Evaluation of Medicinal Product, CPMP _BWP _243_96, London, 1996 and more information concerning biopotency is provided in the last part of this application. In classical incremental dosage desensitisation, where the daily dose of allergen in is increased to a certain maximum, the preferred potency of a dosage unit is from 150 - 1,000,000 SQ-U/dosage unit, more preferred the potency is from 500 - 500,000 SQ-U/dosage unit, more preferred the potency is from 500 - 375,000 SQ-U/dosage unit, more preferably the potency is from 2500 -375,000 SQ-U/dosage unit, more preferred the potency is from 2500 - 250,000 SQ-U/dosage unit, more preferred 25,000 - 250,000 SQ-U/dosage unit, more preferably 25,000 - 125,000 SQ-U/dosage unit, more preferred 25,000 - 100,000 SQ-U/dosage unit and most preferable 25,000 - 75,000 SQ-U/dosage unit.

In another embodiment of the invention the dosage unit is a repeated monodose containing the daily dose of allergen, preferably within the range of from 2500 - 375,000 SQ-U/dosage unit, more preferred 2500 - 250,000 SQ-U/dosage unit, more preferred 25,000 - 250,000 SQ-U/dosage unit, more preferred from 25,000 - 125,000 SQ-U/dosage unit, even more preferred from 25,000 - 100,000 SQ-U/dosage unit, and most preferred from 25,000 - 75,000 SQ-U/dosage unit.

In a particular preferred embodiment the dosage unit comprises a grass allergen extract and the potency is from 150 - 1,000,000 SQ-U/dosage unit, more preferred the potency is from 500 - 500,000 SQ-U/dosage unit, more preferred the potency is from 500 - 375,000 SQ-U/dosage unit, more preferred the potency is from 2500 - 375,000 SQ-U/dosage unit, more preferred the potency is from 2500 - 250,000 SQ-U/dosage unit, more preferred 25,000 - 250,000 SQ-U/dosage unit , more preferred 25,000 - 125,000 SQ-U/dosage unit, more preferred 25,000 - 100,000 SQ-U/dosage unit and most preferred 25,000 - 75,000 SQ-U/dosage unit.

In classical incremental dosage desensitization the potency of a dosage unit according to the invention is from 5 - 50,000 BAU/dosage unit, more preferred the potency is from 15 - 25,000 BAU/dosage unit, more preferred the potency is from about 15 - 17,600 BAU/dosage unit, more preferred the potency is from about 65 - 17,600 BAU/dosage unit, more preferred the potency is from about 65 - 15,000 BAU/dosage unit, more preferred the potency is from about 650 - 15,000 BAU/dosage unit, more preferred 650 - 6,000 BAU/dosage unit, more preferred 650 - 4,700 BAU/dosage unit, most preferable the potency is 650 - 3,500 BAU/dosage unit.

In another embodiment of the invention the dosage unit is a repeated monodose, having a potency of 65 - 17,600 BAU/dosage unit, more preferred 65 - 15,000 BAU/dosage unit, more preferred 650 - 15,000 BAU/dosage unit, more preferred 650 - 6,000 BAU/dosage unit, even more preferred 650 - 4,700 BAU/dosage unit, most preferred 650-3,500 BAU/dosage unit.

In a particular preferred embodiment the dosage unit comprises grass allergen extract and the potency is from 5 - 50,000 BAU/dosage unit, more preferred the potency is from 15 - 25,000 BAU/dosage unit, more preferred the potency is from 15 - 17,600 BAU/dosage unit, more preferred the potency is from 65 - 17,600 BAU/dosage unit, more preferred the potency is from 65 - 15,000 BAU/dosage unit, more preferred the potency is from 650 - 15,000 BAU/dosage unit, more preferred 650-6,000 BAU/dosage unit, more preferred 650 - 4,700 BAU/dosage unit, and most preferred 650 - 3,500 BAU/dosage unit

1 mg allergen extract normally contains between 100,000 and 1,000,000 SQ-unit(s). This means that 1,000,000 SQ are contained in from 1 mg extract to 10 mg allergen extract, and that 100,000 SQ are contained in from 0.1 mg extract to 1 mg allergen extract. In a similar manner, any SQ dose may be transformed into an allergen extract dose range. On this basis, the above dose ranges given in SQ may be recalculated into dose ranges in mg or µg allergen extract, wherein for the lower SQ limit of a range, the lower limit of the corresponding allergen extract range is used, and wherein for the upper SQ limit of a range, the upper limit of the corresponding allergen extract range is used.

Thus, in a further embodiment one dose of allergen consist of 0.15 µg - 10 mg allergen extract, more preferred 0.5 µg - 5 mg, more preferred 0.5 µg - 3.75, more 2.5 µg - 3.75 mg, more preferred 2.5 µg - 2.5 mg, more preferred 25 µg - 2.5 mg, more preferred 25 µg - 1.25 mg even more preferred 25 µg - 1 mg, and most preferred 25 µg - 0.75 mg allergen extract.

In another embodiment of the invention one dose of allergen consist of 2.5 µg - 3.75 mg extract, more preferred 2.5 µg - 2.5 mg allergen extract, more preferred of 25 µg - 2.5 mg extract form, more preferred 1.5 µg - 1.25 mg extract, even more preferred 25 µg - 1 mg extract, most preferred 25 µg - 0.75 mg of extract.

The classification of an allergen as a major allergen can be subject to several tests. An allergen is commonly classified as a major allergen if at least 25% of the patients shows strong IgE binding (score 3) and at least moderate binding (score 2) from 50% of the patients, the binding being determined by an CRIE (Crossed Radio Immune Electrophoresis) (CRIE Strong binding, i.e. visible IgE-binding on an X-ray film after one day; CRIE Moderate binding, i.e. binding after 3 days; CRIE Weak binding, i.e. binding after 10 days). Strong IgE binding from at least 10% of the patients classifies the allergen as an Intermediate allergen and clearly specific binding from less than 10% of the patients classifies it as a Minor allergen. Other methods may also be used in determining the IgE binding of for instance IgE-blots.

In a further embodiment, one dose of allergen has a major allergen content of 0.015 µg - 1 mg, more preferred 0.05 µg - 500 µg, more preferred 0.05 µg - 375 µg, more preferred 0.25 µg - 375 µg, more preferred 0.25 µg - 250 µg, more preferred 2.5 µg - 250 µg, more preferred 2.5 µg - 125 µg, even more preferred 2.5 µg - 100 µg, most preferred 2.5 µg - 75 µg.

In another embodiment of the invention, one dose of allergen has a major allergen content of within the range of 0.25 µg - 375 µg/dosage unit, more preferred of 0.25 µg - 250 µg, more preferred 2.5 µg - 250 µg, more preferred 2.5 µg - 125 µg, even more preferred 2.5 µg - 100 µg, most preferred 2.5 µg - 75 µg/dosage unit.

The content of major allergens may be accounted for by several major allergens depending on the allergen source in question. Normally the number of major allergens is in the range of 1-10, mostly 1-5.

The major allergen may be comprised in an allergen extract or be recombinantly produced. Recombinant major allergens may be used in the same amount as in allergen extracts comprising such major allergen or in higher doses. Higher doses are believed to be more effective, but are also believed to be associated with a risk of potentially more frequent or more severe side effects.

It is believed that for the majority of the average allergic population an effective daily dose of allergen according to the invention will preferably be between 65 BAU/solid dosage unit- 17,600 BAU/solid dosage unit, but a dose as low as 4 and up to 47,000 BAU may be applicable for other allergic patients. Equally hereto, a dose of allergen extract of 0.5 µg - 3.75 mg/dosage unit, or a dose with a major allergen content of 0.05 µg - 375 µg/dosage unit may be suitable for an average allergic individual.

For hypoallergenic variants of major allergens, i.e. allergens with a decreased ability to caused immediate or late phase allergic reactions, a dosage form according to the invention preferably contains 10-100 times more major allergen per dosage form. Such hypoallergenic variants may be of recombinant or natural origin.

Several laboratory tests are available for characterizing an allergen. The most widely used techniques are sodium dodecyl sulphate polyacrylamide gel electrophoresis (SDS-PAGE), isoelectric focusing (IEF), crossed immunoelectrophoresis (CIE) and Rocket Immuno Electrophoresis (RIE). The quantification of individual allergens may be performed by a variety of quantitative immunoelectrophoretic techniques (QIE), Radial Immune Diffusion (RIE) or by enzyme-linked immunosorbent assays (ELISA). The determination of total allergenic activity is most frequently performed by radio allergosorbent test (RAST), Magic Lite assay (LIA) or related techniques. ELISA-based techniques may also be used.

The allergen content of a dosage unit according to the invention can be determined by routine immune assays such as CIE (Cross Immune Electrophoresis), RIE (Radio Immune Electrophoresis) and SDS-PAGE (Sodium Dodecyl Sulphate Poly Acrylamide Gel Electrophoresis) and immune assays such as ELISA and Magic Like Specific IgE assay (LIA).

The pharmaceutical product of the invention comprises one or a plurality of dosage unit(s). The dosage units may be a solid dosage unit, such as a tablet, a lozenge, a capsule and/or a caplet.

The dosage unit may also be a liquid dosage unit, i.e. a volume of a liquid formulation containing one dose of allergen and and up to 50% w/w of the oral daily dose of antihistamine in a human above 12 years of age. According to this embodiment, the pharmaceutical product of the invention may be a container, such as a flask, vial or ampoule comprising one or more liquid dosage units each comprising one dose of allergen and up to 50% w/w of the oral daily dose of antihistamine in a human above 12 years of age. Typically, the flask, vial or ampoule contains a liquid formulation with one dose of allergen and up to 50% w/w of the oral daily dose of antihistamine in a human above 12 years of age.

As mentioned above, one dose of allergen may suitably be the daily dose of allergen.

A liquid formulation is suitably a solution or suspension which may be administered as drops to the oral mucosa, e.g. sublingually, see PCT/DK2006/000609 and WO 05/077410 describing such liquid formulations.

Obvious drawbacks of this dosage form and route of administration is the problems associated with accurate and uniform self administration of the correct dose by the patient (several drops may have to be given, uniformity of the individual drops, application site accuracy, etc.). Additionally, there is a need to refrigerate the drug and include preservatives in the formulation.

The term "solid dosage unit" refers to a dosage unit that is not a liquid when it is administered in the oral cavity, thus "solid dosage unit" refers to e.g. tablet(s) comprising a dose of allergen and an amount of antihistamine as defined above.

In order to ensure that as much as possible of an administered dose of a certain allergen is presented to the mucosa of the oral cavity and additionally that the contact time of the disintegrated product with the mucosa is maximised, it is important that the dosage form disintegrates instantaneously upon contact with the saliva of the oral cavity.

The solid dosage unit is therefore suitably a solid "fast-dispersing dosage unit".

The term "fast-dispersing dosage unit" refers to dosage forms which disintegrate in less than about 90 seconds, preferably in less than about 60 seconds, preferably in less than about 30 seconds, more preferably in less than 20, even more preferably in less than 10 seconds in the human oral cavity, even more preferred in less than 5 seconds, and most preferably in less than 2 seconds after being received in the oral cavity.

In further embodiment of the invention the allergen dosage unit dissolved in saliva is not swallowed until 3 min after administration in order to allow sufficient contact time for e.g. absorption over the mucosal membrane in the mouth.

In yet a further preferred embodiment the allergen dose is not diluted in the oral cavity e.g. by intake of a fluid like water until after 5 min.

The disintegration may for example be measured as described in the European Pharmacopoeia (3rd edition) or the current USP.

Fast dispersing solid dosage forms, which readily release the allergen and antihistamine in the oral cavity are known in the art:

U.S. Patent No 4,371,516 discloses pharmaceutical dosage forms containing active ingredients, which disintegrate rapidly in water. The pharmaceutical dosage forms comprise an open matrix network of carrier material, which disintegrate within 10 seconds.

A freeze-dried fish gelatine based carrier as disclosed in WO 00/61117 is designed to release the active ingredient instantaneously upon contact with saliva when administered in the oral cavity.

A freeze-dried modified starch carrier as disclosed in WO 00/44351 is designed to release the active ingredient instantaneously upon contact with saliva when administered in the oral cavity.

WO 99/21579 discloses a fast-dispersing dosage form comprising a vaccine and an adjuvant for oral use.

WO 02/13858 discloses fast dissolving pharmaceutical composition containing vaccines in the form of a fast dissolving "cake" for oral use. The object of WO 02/13858 appears to be to provide viral or bacterial vaccines that will stay intact in the gastrointestinal tract. This is achieved by protecting the antigen against the acidic content of the stomach by incorporating antacids such as calcium carbonate into the cake.

WO 00/51568 discloses a fast-disintegrating compressed low friability tablet that is designed to dissolve in the mouth in contact with saliva in less than 30 seconds forming an easy-to-swallow suspension.

Any suitable known solid formulation for oromucosal administration, such as sublingual administration, may be used for the dosage unit(s) in the pharmaceutical product of the invention.

A preferred formulation used for the pharmaceutical product of the invention is the non-compressed fast dissolving tablets described in US 2004/0166123.

The advantages of this formulation are described in detail in US 2004/0166123 and include therapeutic effect without an adjuvant, stability, low friability, etc. Methods for measuring stability, friability, ect. are also described in this patent application.

The term "non-compressed" refers to a solid dosage unit, which is manufactured by removal of a liquid from a solidified system comprising matrix forming agents, active ingredient and other suitable ingredients resulting in an allergen and antihistamine comprising solid matrix.

The non-compressed fast-dispersing solid dosage forms described in US 2004/066123, which may be manufactured by removal of a liquid from a solidified system comprising matrix forming agents, active ingredient and other optional agents, is preferably manufactured *in situ.* The *in situ* manufacturing process generally involves removal of solvent from a solidified system of the active ingredients and the matrix forming excipients within the final container such as a blister pack.

More precisely, the fast-dispersing solid dosage form according to the invention can be prepared by a sublimation process according to the process disclosed in U.S. Patent No. 4, 371,516. Accordingly, a solidified solution of the allergen, antihistamine and the matrix forming excipients is subjected to sublimation. The sublimation process is preferably carried out by freeze-drying the solution. The solution is contained in a depression of the multi dosage container during the freeze-drying step to produce a solid form in any desired shape. The multi dosage container can be cooled using liquid nitrogen or solid carbon dioxide. After the freezing step the frozen solution in the multi dosage container is subjected to reduced pressure and, if desired, controlled application of heat to aid the sublimation of the solvent.

One type of pharmaceutically acceptable excipients in the fast-dispersing solid dosage unit according to invention are matrix forming agents.

The pharmaceutical product of the invention may also comprise excpients such as antacids, diluents, enhancers, mucoadhesive agents, flavouring agents, taste masking agents, preservatives, antioxidants, surfactants, viscosity enhancers, colouring agents, pH modifiers, sweeteners etc. These excipients are all selected in accordance with conventional pharmaceutical practice in a manner understood by persons skilled in the art of formulating allergen therapeutics.

Matrix forming agents suitable for use according to the present invention include excipients derived from animal or vegetable proteins such as gelatines, dextrins and soy, wheat and psyllium seed proteins; gums such as acacia, guar, agar and xanthan; polysaccharides; starch and modified starch, alignates; carboxymethylcellulose; carrageenans; dextrans; pectins; synthetic polymers such as polyvinylpyrrolidone; and polypeptide/protein or polysaccharide complexes such as gelatine-acacia complexes. Gelatines are a heterogeneous mixture of water soluble colloid macromolecules. Such heterogeneous mixtures of average molecular weights distribution may be obtained from hydrolytic action on collagen rich material of animal origin such as bone, skin, tendons, ligaments etc. Gelatines may be derived from mammal e.g. cattle, pig or non-mammals e.g. warm or cold-water fish. Gelatines can be hydrolysed or non-hydrolysed, cross-linked or non-cross-linked. They can further be of a gelling or non-gelling type, the non-gelling type typically being derived from coldwater fish. In another particular embodiment starch is used. Starches are complex mixtures of carbohydrate polymers.

Other matrix forming agents suitable for use according to the present invention include sugars such as mannitol, dextrose, sucrose, lactose, galactose and trehalose; dextran, cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

The formulations of the invention may also be one of the formulations described in patent application No. PCT/DK2006/000546, which relates to spray freeze dried compositions comprising allergen.

The solid dosage unit preferably comprises at least about 50% w/w of at least one matrix forming agent of the dosing solution. The term "dosing solution" as used in this context means a non-solid volume of a formulation of the matrix forming agents, the allergen, antihistamine and possibly other excipients that is prepared before the solidification step.

The dosing solution for forming the solid dosage form comprises about 5-30% w/w, more preferably about 5-20% w/w, even more preferred between about 5-12% w/w of at least one matrix forming agent.

The need for dry matter content of the dosing solution will also depend on the dimensions of the tablet. Preferably the solid dosage units according to the present invention have a diameter between about 3 to about 30 mm, more preferably between about 5 to about 20 mm. Preferably the solid dosage units according to the present invention have a weight between about 1 to about 100 mg, more preferably between about 10 to about 50 mg, most preferably between about 25 to about 35 mg. Preferably the solid dosage units according to the invention have a height between about 0.5 to about 7.5 mm, more preferably between about 1 to about 5 mm.

A fast-dispersing solid dosage unit comprising fish gelatine and mannitol as matrix-forming excipients has been found to be especially advantageous with respect to stability, visual appearance, low friability, tensile strength, peak load to fracture and mouth feel. In a preferred embodiment the fast-dispersing solid dosage units comprises a solid network of the allergen, antihistamine and matrix forming agents in the form of fish gelatine and mannitol. In order to obtain a solid network, the ratio of fish gelatine to mannitol should be controlled. In a preferred embodiment the ratio of fish gelatine to mannitol is from about 2:20 to about 20:1, more preferably from about 2:10 to about 10:1, most preferably from about 3:5.5 to about 6.5:3.

In yet a further embodiment the ratio of fish gelatine to mannitol is 4:3.

In another embodiment the ratio of fish gelatine to mannitol is 6.5:5.5.

In a further embodiment the ratio of fish gelatine to mannitol is 6.0:5.08.

The solid dosage unit according to the present invention may be manufactured from a dosing solution, which is first frozen and then freeze dried. In a preferred embodiment the content of fish gelatine is between about 2-20% w/w of the dosing solution and the content of mannitol is between about 1-20% w/w of the dosing solution. In another preferred embodiment the content of fish gelatine is between about 2-10% w/w of the dosing solution and the content of mannitol is between about 1-10% w/w of the dosing solution. In a further preferred embodiment the content of fish gelatine is between about 3-6.5% w/w of the dosing solution and the mannitol is between about 3-5.5% w/w of the dosing solution.

In yet a further embodiment the matrix comprises about 4% w/w fish gelatine in the dosing solution and about 3% w/w mannitol in the dosing solution

In another embodiment the matrix comprises about 6.5% w/w fish gelatine in the dosing solution and about 5.5% w/w mannitol in the dosing solution.

In a further embodiment the matrix comprises 6.0% w/w fish gelatine in the dosing solution and 5.08% w/w mannitol in the dosing solution.

A fast-dispersing solid dosage form comprising starch and mannitol as matrix-forming excipients has also been found to be especially advantageous with respect to stability, visual appearance, low friability, tensile strength, peak load to fracture and mouth feel. In a preferred embodiment the fast-dispersing solid dosage forms comprises a solid network of the allergen, the antihistamine, and matrix forming agents in the form of starch preferably pre-gelatinised from e.g. potato, wheat, maize, corn or rice and mannitol. In order to obtain a solid network, the ratio of starch to mannitol should be controlled. In a preferred embodiment the ratio of starch to mannitol is from about 2:20 to about 20:1, more preferably from about 2:10 to about 10:1, most preferably from about 3:5.5 to about 6.5:3.

In yet a further embodiment the ratio of starch to mannitol is 1:1.

The solid dosage unit according to the present invention is manufactured from a dosing solution, which is first frozen and then freeze dried. In a preferred embodiment the content of starch is between about 2-20% w/w of the dosing solution and the content of mannitol is between about 1-20% w/w of the dosing solution. In another preferred embodiment the content of starch is between about 2-10% w/w of the dosing solution and the content of mannitol is between about 1-10% w/w of the dosing solution. In yet a further preferred embodiment the content of starch is between about 3-6.5% w/w of the dosing solution and the mannitol is between about 3-5.5% w/w of the dosing solution

In another embodiment the matrix comprises about 4.4.% w/w starch of the dosing solution and about 4.4% w/w mannitol of the dosing solution.

Preferably matrix compositions containing an allergen extract should be adjusted to pH between 3.5-10, more preferably 4-9, most preferably 6-9.

Preferably the ionic strength of the extract of 10 µg/ml is in between 1-1500 µS/cm (S=Simens), more preferred between 300 - 800 µS/cm, most prefered about 500 µS/cm, for a matrix and allergen containing system it is preferred that the ionic strength is between 1-2000 µS/cm, more preferred about 500-1500 µS/cm.

Solid dosage units according to the invention may further comprise colouring agents, flavours, pH modifiers, sweeteners or taste-masking agents. Suitable colouring agents include red, black and yellow iron oxides and FD & C dyes such as FD & C blue No. 2 and FD & C red No. 40. Suitable flavouring agents include mint, raspberry, liquorice, orange, lemon, grapefruit, caramel, vanilla, cherry and grape flavours and combination of these. Suitable pH modifiers include citric acid, tartaric acid, phosphoric acid, hydrochloric acid and maleic acid. Suitable sweeteners include aspartame, acesulfame K and thaumatic. Suitable taste-masking agents include sodium bicarbonate, ion-exchange resins, cyclodextrin inclusion compounds, adsorbates or microencapsulated actives.

Adjuvants are sometimes used to enhance the absorption of the allergen as well as to enhance the immune-stimulating properties of the allergen.

In one preferred embodiment of the invention the fast-dispersing solid dosage unit according to the invention does not comprise an adjuvant.

In another embodiment of the invention at least one adjuvant is incorporated into the dosage unit according to the invention. Examples of suitable adjuvants are aluminium salts, aluminium hydroxide such as Alhydrogel^{®}, non-toxic bacterial fragments, cytokines, cholera toxin (and detoxified fractions thereof), cholera toxin subunit b, chitosan, homologous heat-labile fragments of E.coli (and detoxified fractions thereof), saponins, bacterial products such as lipopoly-saccharides (LPS) and muramyl dipeptide (MDP), liposomes, CpG (immunostimulatory DNA sequences), lactide/glycolide homo ± copolymers in the form of microparticular polymers etc. The use of adjuvants in allergen pharmaceutical product e.g. vaccines are often reasoned by the fact the allergens in question are not able to penetrate the barrier to be passed. The adjuvants thus may serve as absorption enhancing agents or they may act as immunostimulants. The use of adjuvants may, however, be associated with serious draw backs such as unintended stimulation of various mechanisms of the immune response, systemic lupus erythematosus or affecting the barrier capabilities of the mucosal membranes and thus allowing the passage of hazardous substances. Further from an industrial point of view addition of an adjuvant further constitute further manufacturing and material cost besides the large demand for documentation in respect to drug registration.

A non-compressed fast-dispersing solid dosage unit may be mucoadhesive to some extent. However in a preferred embodiment of the invention, it may be necessary to further add mucoadhesive excipients to said dosage unit in order to increase the contact time of the dosage form with the mucosa of the oral cavity. Suitable mucoadhesive excipients are polyacrylic polymers such as carbomer and carbomer derivatives; cellulose derivatives such as hydroxypropylmethylcellulose, hydroxyethylcellulose, hydroxypropylcelllulose and sodium carboxymethylcellulose; natural polymers such as gelatine, sodium alginate, pectin and glycerol.

The solid dosage unit(s) according to the invention may be manufactured and packed in disposable containers containing a plurality of solid dosage units i.e. multi dosage containers. The methods and materials as described in U.S. Patent Nos. 5,729,958 and 5,343,762 are particularly favoured. Examples of suitable multi dosage containers are All Aluminium Blister packs, blister packs made of polymers e.g. polypropylene, blister packs of PVC and blister packs formed from PVC/PVdC laminate and sealed with e.g. aluminium laminated to calendered kraft paper, Aclar® or Triplex®.

In an embodiment the dosage unit is manufactured and packed in blister packs formed from PVC/PVdC laminate and sealed with aluminium laminated to calendered kraft paper. In another embodiment hereof the blister pack are enclosed in an aluminium sachet of suitable size, composed of aluminium laminated to calendered kraft paper.

In yet another embodiment the solid dosage unit(s) are packed in blister packs formed from aluminium and sealed with aluminium laminated to calendered kraft paper.

In a further embodiment the solid dosage unit is packed in multilamilar blister packs formed from e.g. five layer aluminium laminate and sealed with aluminium laminated to calendered kraft paper.

In yet another embodiment the solid dosage unit is packed in blister packs formed from aluminium laminate and sealed with aluminium laminated to calendered kraft paper in such a way that is difficult for children to open the blister pack e.g. child resistant packs.

In order to ensure that allergen containing residues from the solid dosage unit is not released to the environment upon opening the multi dosage container, it is important that the friability of the dosage form is as low as possible without jeopardizing the allergen release from the dosage form following oral administration. Acceptable levels and methods for measuring friablility are described in US 2004/055123, see in particular paragraph [0191] to [0195].

In one embodiment each of the solid dosage units of a treatment pack are located in individually sealed blisters in a multiple blister pack.

The pharmaceutical product of the invention may thus be a multi dosage container which may contain any conceivable number of solid dosage units. Preferably, the solid allergen dosage units are packaged and used as a group. Individual solid dosage units are packaged by dispensing as a liquid mixture into individual containers, followed by removal of water. Multiple blisters may be arranged in larger sheets and multiple sheets may be packaged and sold together. For example, a container such as a blister pack may comprise a plurality of solid dosage units preferably 1-100 solid dosage units, more preferred 1-30, more preferred 1-20, and most preferred 1-10 solid dosage units per blister pack.

In another embodiment the pharmaceutical product is a treatment pack for the treatment of allergy or alleviating symptoms of allergy. The treatment pack comprises a sealed package of multiple solid dosage units, each of which comprises a dose of an allergen combined with antihistamine. The treatment pack may contain for example, at least 2, 4, 6, 7, 10, 14, 20, 30, 60, 90, 100, 120, 150, 200, 240, or more solid dosage units. The treatment pack may contain enough dosage units for an entire treatment, or enough for a portion of a treatment. Advantageously the treatment pack contains at least one month's supply of unit doses.

In another advantageous embodiment of the invention, all solid dosage units of a treatment pack contain the same allergen dosage and the same amount of antihistamine, thus removing the necessity for manufacturing, distributing, and storing multiple dosage units for treatment of a single allergy in an individual.

Most desirably, the multiple solid dosage units of a treatment pack have been formed from a liquid mixture by an in situ process that removes a suspension and/or solvation liquid, which may comprise water and/or other organic solvents and are fast dispersing.

Treatment of in particular seasonal allergies such as hay fever is normally associated a particular time of year were exposure to the offending allergen is present or elevated. The allergen season is will vary with the allergen source e.g. the pollen and the climatic conditions for the allergen source in the particular territory. Thus, the season for an allergen will differ in one part of the world from another part of the world depending on the climate, but will normally fall within the same period of the year for the same territory varying with the actual conditions of that year (see for instance "Aerobiology and inhalant allergies", Chapter 19, T.A.E. Platts-Mills & W.R. Solomon (Ed. S. Manning) 1993, Mosby-Year Book, St. Louis). This will be well known to a skilled person when a season is normally expected to start for a particular allergen in a particular region.

In one embodiment of the invention a methods of treatment is provided including a pre-seasonal treatment i.e. an administration of solid dosage units according to the invention before the allergen season. In a particular preferred embodiment the pre-seasonal treatment period comprises administration of solid dosage units according to the invention for a period of more than 2 weeks prior to the allergen season, more preferably between 4-20 weeks, most preferably between 8-12 weeks.

In one embodiment, the invention provides a treatment regimen that utilizes a single dose of allergen and a single dose of antihistamine for completing an entire desensitization treatment, without having to updose i.e. increase with the level of allergen until the maintenance dose is reached. This embodiment is advantageous because it simplifies and economizes on dosage form manufacture, distribution and storage by not requiring multiple dose quantities for a single treatment. Moreover, by simplifying a treatment course, patient compliance is improved, which directly leads to greater clinical effectiveness.

The invention also provides a method for treating a mammal afflicted with allergy, comprising repeated oromucosal administration of dosage units until the itching symptoms are expected to have disappeared or reduced considerably, e.g. repeated administration in 10, 20 , 30, 40 , 50, 60 days or more than 150 days.

In one embodiment the mammal is treated by repeated oromucosal administration of dosage units of the invention in a period of up to 30 days, preferably up to 20 days or more preferred up to 10 days.

Suitably one dosage unit comprises a daily dose of allergen and an amount of antihistamine as described above.

A first period of treatment with a dosage unit comprising one dose of allergen and antihistamine, e.g. 10, 20, 30, 40, 50, 60 days or more than 150 days, may followed by a period up of to 5 years or until the allergy symptoms are relieved, of administration of dosage units comprising the same daily dose of allergen but without the antihistamine.

The oromucosal administration is sublingual administration.

The invention also relates the use of (a) an allergen, (b) an antihistamine and (c) pharmaceutically acceptable excipient(s) for the preparation of a pharmaceutical product as above comprising one or a plurality of dosage unit(s) for the treatment of allergy or alleviating symptoms of allergy and reduce itching in the oral cavity caused by the administration of allergen.

In one embodiment, the invention relates to the use of (a) an allergen, (b) an antihistamine and (c) pharmaceutically acceptable excipients for the preparation of a pharmaceutical product comprising a plurality of dosage units and where the number of dosage units is the number required for oromucosal administration in a period of up to 30 days, more preferred in up to 20 days and most preferred in up to 10 days. Suitably one dosage unit comprises the daily dose of allergen.

The invention also relates to a pharmaceutical product according to the invention for the treatment of allergy or alleviating symptoms of allergy and reduce itching in the oral cavity caused by the administration of allergen.

The invention also relates to a pharmaceutical product according to the invention_comprising a plurality of dosage units and where the number of dosage units is the number required for oromucosal administration in a period of up to 30 days, more preferred in up to 20 days and most preferred in up to 10 days. Suitably one dosage unit comprises the daily dose of allergen and an amount of antihistamine as described above.

The oromucosal administration is sublingual administration.

### Definitions

The term "pharmaceutically acceptable excipient" refers to any pharmaceutically acceptable ingredient that may be added to the formulation besides the active ingredient.

The term "matrix forming agent" refers to any pharmaceutically acceptable water-soluble or water-dispersible excipient that will serve as a carrier for the active ingredient in the solid dosage form.

The term "oromucosal administration" refers to a route of administration where the dosage form is placed under the tongue or anywhere else in the oral cavity to allow the active ingredient to come in contact with the mucosa of the oral cavity or the pharynx of the patient in order to obtain a local or systemic effect of the active ingredient. An example of an oromucosal administration route is sublingual administration.

The term "sublingual administration" refers to a route of administration, where a dosage form is placed underneath the tongue in order to obtain a local or systemic effect of the active ingredient.

The term "allergen" refers to any naturally occurring protein or mixtures of proteins that have been reported to induce allergic, i.e. IgE mediated reactions in an individual upon their repeated exposure to the protein. The allergen may be used in the form of an allergen extract, a purified allergen, a modified allergen or a recombinant allergen or a recombinant mutant allergen, any allergen fragment above 30 amino acids or any combination thereof.

The expression "extract" as used commensurate with allergen, refers to an extract obtained by extraction of a biological allergen source material as generally described in "Allergenic extracts", H. Ipsen et al, chapter 20 in Allergy, principle and practise (Ed. S. Manning) 1993, Mosby-Year Book, St. Louis. Such extract may be obtained by aqueous extraction of water soluble material followed by purification steps like filtration to obtain the solution i.e. the extract. Generally, an allergen extract comprises a mixture of proteins and other molecules. Allergen proteins are often classified as a major allergen, an intermediate allergen, a minor allergen or no classification. An allergen extract generally comprises both major and minor allergens. Major allergens will generally constitute approximately 5-15% of an average allergen extract, more often about 10%. Classification of an allergen is based on an assessment of the clinical importance of the particular allergen. Examples of important major allergen found in an extract include grass group 1 and 5 and 6 allergens (e.g. Phl p 1, 5, and 6), dust mite group 1 and 2 allergens (e.g. Der p 1 , Der p 2), tree pollen allergen 1 (Bet v 1), cedar pollen allergen 1 and 2 (e.g. Cry j 1, Cry j 2), ragweed pollen 1 and 2 (Amb a 1, Amb a 2), cat allergen 1 (i.e. Fel d1). The average allergic person will be sensitised to and react to one or more major allergens and further may also be sensitised and react to minor allergens.

Amounts of allergen extract referred to herein refers to the dry matter content of such allergen extracts.

Preferably the water content of the dry matter does not exceed 10%, more preferably 5% by weight.

The expression "biological allergen source material" as used therein refers to any biological material comprising one or more allergens. Examples of such materials are acarids PMB (Pure Mite Body) or WMC (Whole Mite Culture), defatted or non-defatted pollens from e.g. grasses, herbs, weeds and trees, animal hair and dander, pelt, fungi mycelia and spores, insect bodies, venom or saliva and foods.

Biological allergen source materials may comprise contaminating materials, such as foreign pollen and plant and flower debris for an allergen pollen source material.

The degree of contamination should be minimized. Preferably, the content of contaminants should not exceed 10% (w/w) of the biological source material.

Normally an allergen extract contains at least 10% protein of the dry matter content of the allergen extract as determined in a standard protein assay such as BCA or Lowry and the remainder consists of other "non-protein material," which may be components such as lipids, carbohydrates, or bound water which originate from the biological allergen source.

An allergen extract may be formulated and stored in form of a freeze-dried material obtainable by freeze-drying a liquid allergen extract at a pressure of below 800 micro bar and for a period of up till 100 hours removing the water.

In the field of allergy extracts, there is no international accepted standardization method. A number of different units of extract strength i.e. bio-potency exist. The methods employed and the units used normally measure the allergen content and biological activity. Examples hereof are SQ-Units (Standardized Quality units), BAU (Biological Allergen Units), BU (biological units), UM (Units of Mass), IU (Intemational Units) and IR (Index of Reactivity). Hence, if extracts of different origins are used, they need to be standardized against a reference extract in order to determine their potency in SQ units or any of the above mentioned units. The subject matter is dealt with in "Allergenic extracts", H. Ipsen et al, chapter 20 in Allergy, principle and practice (Ed. S. Manning) 1993, Mosby-Year Book, St. Louis and Løwenstein H. (1980) Arb Paul Ehrlich Inst 75:122.

The bio-potency, i.e. the in vivo allergenic activity, of a given extract depends on a number of factors, the most important being the content of major allergens in the extract, which varies with the composition of the biological source material.

The amount of allergen extract in grams to be used for obtaining a desired bio-potency varies with the type of extract in question, and for a given type of extract the amount of allergen extract varies from one batch to another with the actual bio-potency of the extract.

For a given batch of extract, the amount of allergen extract in grams to be used for obtaining a desired bio-potency may be determined using the following procedure:
a) The bio-potency of various amounts of a reference extract is determined using one or more immunological in vivo tests to establish a relationship between bio-potency and amount of reference extract. Examples of the said immunological in vivo tests are Skin Prick Test (SPT), Conjunctival Provocation Test (CPT), Bronchial Challenge with Allergen (BCA) and various clinical trials in which one or more allergy symptoms is monitored, see for example e.g. Haugaard et al., J Allergy Clin Immunol, Vol. 91, No. 3, pp 709-722, March 1993.
b) On the basis of the established relationship between bio-potency and reference extract, the bio-potency of one or more relevant doses for use in the dosage units of the invention is selected with due consideration to a balance of the factors of i) the effect of treating or alleviating symptoms of allergy, ii) side effects recorded in the immunological in vivo tests, and iii) the variability of i) and ii) from one individual to another. The balancing is done to obtain a maximal adequate therapeutic effect without experiencing an unacceptable level of side effect. The way of balancing the factors are well known to those skilled in the art
   The bio-potency of the one or more relevant doses found may be expressed in any biopotency unit available, such as SQ units, BAU, IR units, IU, cf. above.
c) From the reference extract one or more bio-potency reference standard extracts is prepared and, if used, the bio-potency unit values of the reference standard extracts are calculated on the basis of the bio-potency unit value allocated to the one or more relevant doses, e.g. such a standard for BAU can be obtained from FDA as illustrated below.
d) For the reference standard extracts of each extract type, a number of parameters for evaluating the bio-potency of extracts are selected. Examples of such evaluation parameters are total allergenic activity, the amount of defined major allergens and overall molecular composition of the extract. The total allergenic activity may be measured using an in vitro competitive immunoassay, such as ELISA and MagicLite® luminescence immunoassay (LIA), using a standardised antibody mixture raised against the extract obtained using standard methods, e.g. antibodies raised in mouse or rabbit, or a pool of allergic patients sera. The content of major allergens may e.g. be quantified by rocket immuno-electrophoresis (RIE) and compared to the reference standards. The overall molecular composition may be examined using e.g. crossed immunoelectrophoresis (CIE) and sodium dodecyl sulphate polyacrylamide gel electrophoresis (SDS-PAGE).
e) For a given batch of extract of unknown bio-potency (test extract), the amount of extract to be used for obtaining a desired bio-potency level (effective dose for use in the dosage unit according to the present invention) may be determined as follows: For each evaluation parameter selected, the test extract is compared with the reference standard extracts using the relevant measurement methods as described above, and on the basis of the measurement results the amount of extract having the desired bio-potency is calculated.

SQ-Unit: The SQ-Unit is determined in accordance with ALK-Abelló A/S's "SQ biopotency"-standardisation method, where 100,000 SQ units equal the standard subcutaneous maintenance dose. Normally 1 mg of extract contains between 100,000 and 1,000,000 SQ-Units, depending on the allergen source from which they originate and the manufacturing process used. The precise allergen amount can be determined by means of immunoassay i.e. total major allergen content and total allergen activity. As the pharmaceutical products of the invention contain an amount of antihistamine which is effective in relieving one of the side-effects (itching in the mouth) the amount of allergen in the dosage units of the invention may in some cases be higher that if the antihistamine was not present.

BAU (Biological Allergen Units) is biological potency units as determined according to the requirements of the FDA for allergen product described in "Quantitative determination of relative potency of allergenic extracts" ("Methods of the allergen products testing Laboratory" "ELISA competition assay". Page 15, #49N-0012, FDA, October 1993). A dose of 100,000 SQ-Units containing grass extract equals a content of 2600-4700 BAU according to the method above. Likewise, other extracts can be assessed according to the method above.

The term "effective dose of an allergen" shall mean a dose which when taken once or repeatedly in a monodose or in incremental doses results in, for example, an adaptive immune response and thus serves as means to desensitise allergic patients. Preferably, the term shall mean the amount of allergen in each dosage unit necessary to induce an adaptive immune response after repeated administration of said solid dosage units in accordance with a treatment regimen (over a period ranging from a few administrations to at least one daily administration over several months). Preferably desensitization includes the alleviation of allergic symptoms upon administration of the dose. Clinical allergy symptoms include rhinitis, conjunctivitis, asthma, urticaria, eczema, which includes reactions in the skin, eyes, nose, upper and lower airways with common symptoms such as redness and itching of eyes and nose, itching and runny nose, coaching, weezing, shortness of breathe, itching, and swelling of tissue.

In a preferred embodiment a pharmaceutical product according to the present invention has a profile where one or more of the following immunological changes can be found; an increased allergen specific IgG response, an increased allergen specific IgA response, reduced allergen specific IgE response, few local side effects; reduced allergen specific effector responses of eosinophils, basophils, lymphocytes and/or monocytes; induction of T cells with regulatory potential, increased ratio of allergen specific Th1 cells to allergen specific Th2 cells, induction of other cells with regulatory potential, reduced allergen specific Th2 response.

The invention is illustrated in the examples below.

### Example 1

A dosing solution of an allergen vaccines containing Phleum pratense grass pollen extract (0.0047 mg extract, 0.047 mg extract and 0.235 mg extract and desloratidine 2.5 mg, 1.25 mg, 0.5 mg) may suitably have the following composition:

**Table 1 API=Active Pharmaceutical Ingredient**

| **Ingredients** | **Unit** | **Dosage form 1** | **Dosage form 2** | **Dosage form 3** | **Function** |
|---|---|---|---|---|---|
| **Drug substance:** | | | | | API |
| *Phleum pratense* | SQ-U | 2500 | 25000 | 125000 | |
| | mg extract | 0.0047 | 0.047 | 0.235 | |
| **Antihistamine: desloratidine** | mg | 0.5 | 1.25 | 2.5 | API |
| **Other ingredients** | | | | | |
| Purfied water | mg | q.s to 250 mg | q.s to 250 mg | q.s to 250 mg | solvent |
| Gelatine (standard molecular weight fish gelatin, Croda UK) | mg | 10 | 10 | 10 | Matrix |
| Mannitol | mg | 7.5 | 7.5 | 7.5 | Matrix |
| Sodium hydroxide | mg | q.s | q.s | q.s | pH adjustment to 7.5 |

### Grass extract

Grass pollen extract may be prepared according to the method described in Ipsen and Løwensten (1983) Jour. Allergy. Clin. Immunol. 72:2, page 150-159. In short grass pollen is extracted in water containing ammonium hydrogen carbonate, for 20 hours at 5°C. Particulate matter is removed by centrifugation and the supernatant is dialysed against water (3 times), lyophilised and stored cold until reconstitution.

### Solid dosage unit:

### Non-compressed tablets may be prepared as follows:

1. The mannitol is added to an aliquot of the purified water (not less than 50% of the total batch requirement) and allowed to dissolve.
2. The gelatine is added to the mannitol solution and the solution is stirred on a magnetic stirrer until the gelatine is fully dissolved.
3. A second aliquot of the purified water (not more than 35% of the total batch requirement) is used to reconstitute the allergen extract in the vials. The reconstituted allergen extract and the antihistamine is added to the mannitol-gelatine solution.
4. The pH of the bulk formulation is adjusted to pH 7.5 using freshly prepared sodium hydroxide solution (3% w/w).
5. The additional amount of purified water required to complete the formulation is calculated and transferred to the bulk mix.
6. The solution is dosed into pre-formed blister packs. The solutions is dosed under ambient temperature conditions.
7. After dosing, the filled blister packs is passed through a liquid nitrogen freeze tunnel. All frozen products were immediately placed in a frozen storage, prior to freeze-drying. The dosage units are freeze-dried using standard conditions of shelf temperature and chamber pressure.
8. The freeze dried units are sealed with a lidding foil and finally packed in a sachet

Antihistamine may be added in any of steps 1-5, but is preferably added in step 3.

The solid dosage unit prepared by this method will suitably have an average weight of 18 mg and average diameter of 11 mm.

### Example 2.

A dosing solution of an allergen vaccine containing Phleum pratense grass pollen extract (0,0047, 0,047 and 0,235 mg and 0.5 mg, 1.25 mg and 2.5 mg levocetirizine) and starch may suitably have the following composition:

**Table 2:**

| **Ingredients** | **Unit** | **Dosage form 1** | **Dosage form 2** | **Dosage form 3** | **Function** |
|---|---|---|---|---|---|
| **Drug substance:** | | | | | |
| *Phleum pratense* | SQ-U | 2500 | 25000 | 125000 | API |
| | mg extract | 0.0047 | 0.047 | 0.235 | |
| **Antihistamine: Levocitirizine** | mg | 0.5 | 1.25 | 2.5 | API |
| **Other ingredients** | | | | | solvent |
| Purified water | Mg | q.s to 250 mg | q.s to 250 mg | q.s to 250 mg | |
| Pre-gelatinised starch | Mg | 8mg | 9mg | 11mg | Matrix |
| Mannitol | Mg | 8mg | 9mg | 11mg | Matrix |
| Sodium hydroxide | Mg | q.s | q.s | q.s | pH adjustment to 7.5 |

Manufacturing process for the non-compressed solid dosage unit:
Same as example 1, pre-gelatinised starch is added instead of gelatine (fish source). The solid dosage will suitably have an average weight of 19 mg and average diameter of 11 mm.

### Example 3

A dosing solution of an allergen vaccines containing Phleum pratense grass pollen extract (0.0047 mg extract, 0.047 mg extract and 0.141 mg extract and 2 mg desloratidine, 0.95 mg desloratidine, 0.25 mg desloratidine) may suitably have the following composition:

**Table 3:**

| **Ingredients** | **Unit** | **Dosage form 1** | **Dosage form 2** | **Dosage form 3** | **Function** |
|---|---|---|---|---|---|
| **Active substance:** | | | | | Active substance |
| *Phleum pratense* | SQ-U | 2500 | 25000 | 75000 | |
| | mg extract | 0.0047 | 0.047 | 0.141 | |
| **Antihistamine: desloratidine** | mg | 0.25 | 0.95 | 2.5 | Active substance |
| **Other ingredients** | | | | | Solvent |
| Purified water | mg | q.s. to 250 mg | q.s. to 250 mg | q.s. to 250 mg | |
| Gelatine (fish source)* | mg | 16 | 16 | 16 | Matrix |
| Mannitol | mg | 14 | 14 | 14 | Matrix |
| Sodium hydroxide | mg | q.s. | q.s. | q.s. | pH adjustment 7.5 |

### Example 4

Solid allergen vaccine dosage units containing varying ratios of matrix forming agents may be prepared.

The solid dosage unit may contain 75,000 SQ-U phleum pratense grass pollen extract and 2.5 mg, 2 mg, 1.25 mg, 0.5 mg, 0.25 mg or 0.05 mg desloratidine or levocitirizine. The ratio of matrix forming components in the dosing solution could be as in table 4:

| **% gelatin** | **% mannitol** |
|---|---|
| 4.00 | 3.00 |
| 4.00 | 3.00 |
| 5.00 | 3.75 |
| 5.00 | 3.75 |
| 6.00 | 4.50 |
| 6.00 | 4.50 |
| 7.00 | 5.25 |
| 7.00 | 5.25 |

The dosage units will typically have a unit diameter of 12 mm as described previously

The solid dosage unit may contain 75,000 SQ-U phleum pratense grass pollen extract, fish gelatine and Mannitol and 2.5 mg, 2 mg, 1.25 mg, 0.5 mg, 0.25 mg or 0.05 mg desloratidine or levocetirizine. The ratio of matrix forming components in the dosing solution could be as in table 5:

| **% gelatin** | **% mannitol** |
|---|---|
| 5 | 4 |
| 6.5 | 5 |
| 6.5 | 5.5 |
| 5 | 7 |
| 8 | 4 |
| 8 | 7 |
| 7 | 7 |
| 7 | 5 |
| 6 | 4 |
| 6 | 7 |

The dosage units will typically have a unit diameter of 12 mm as described previously.

### Example 5

Allergen vaccine containing *Phleum* ***pratense*** pollen extract

### Composition of dosage solution

**Table 6:**

| **Ingredients** | **Composition % w/w** | **Dosage form 2 25,000** | **Dosage form 3 75,000** |
|---|---|---|---|
| Gelatin Fish (Norland, Canada) | 6.0 | 14 mg | 14 mg |
| Mannitol | 5.08 | 12.7 mg | 12.7 mg |
| API (grass pollen extract) | | 25,000 SQ-U | 75,000 SQ-U |
| API (desloratidine) | | 2 mg | 2 mg |
| NaOH | qs to pH 7.5 | qs to pH 7.5 | qs to pH 7.5 |
| Purified water | qs to 250 mg | qs to 250 mg | qs to 250 mg |
| Total % /Wet fill weight | 100% | 250 mg | 250 mg |
| Dried weight | | 27.7 mg | 27.7 mg |

The formulation may be manufactured as described in example 1 and the solid dosage form will have an average diameter of 13 mm.

### Example 6

Investigation of the effect of desloratidine on adverse effects such itching in or around the mouth and the lips, ithching throat, itching in the ears and eyes caused by Phleum pratense pollen extract

The study was is was a cross-over, single-centre investigation.

### Recruitment of patients:

The patients participated on two single days (visit 1 and visit 2) separated by approximately two weeks to avoid development of tolerance due to Grazax treatment. The subjects received one Grazax 75,000 SQ-T on Visit 1 and if they reported treatment related local adverse events in the mouth and/or throat after intake they were eligible for Visit 2. If no treatment related adverse events were reported after Grazax intake on Visit 1 the subjects were excluded from the study.
On visit 2, the subjects received 1 antihistamine (Aerius (Desloratadine) melting tablet 2.5 mg, i.e. orally-disintegrating tablet). Grazax was administered immediately after intake of antihistamine.

AEs were reported on AE pages. AEs were rated mild, moderate or severe. Furthermore, the subjects rated the AE severity on a Visual Analogue Scale (VAS). The AEs of interest were itching mouth, itching throat or other local reactions.

### Study Population

A total of 15 subjects attended visit 1. From these 15, 6 subjects reported treatment related adverse events. These were therefore eligible for visit 2. Key selection criteria were:
1. A clinical history of grass pollen induced allergic rhinoconjunctivitis of two years or more requiring treatment during the grass pollen season
2. Reporting of treatment related allergic adverse events (local reactions in mouth and throat) after intake of Grazax at screening (visit 1).
3. No uncontrolled asthma in the past 12 months (GINA GUIDE 2006; Uncontrolled means three or more of the following features in the same week; Daytime symptoms, need for reliever /rescue medication, limitation of normal activities, nocturnal symptoms / awakenings)
4. FEV₁ < 70% of predicted value
5. No clinical history of symptomatic seasonal allergic rhinitis and/or asthma due to another allergen which may cause symptoms during the conduct of this trial
6. No history of emergency visit or admission for asthma in the previous 12 months
7. No use of an investigational drug within 30 days prior to screening
8. No history of angioedema and anaphylaxis, including anaphylactic food allergy, insect venom anaphylaxis, exercise anaphylaxis or drug induced anaphylaxis

### Visit Schedule

### Visit 1

The following procedures were performed:
- Ask for allergy symptoms - patients who reported allergy symptoms from e.g. grass pollen, tree, weed pollens etc. before administration of Grazax were not allowed to participate
- Intake of Grazax and reporting of possible AEs with a subsequent 60 minutes observation period
- Rating of AEs on Visual Analogue Scale
- Inclusion/exclusion criteria

### Visit 2

The following procedures were performed (approximately 14 days after visit 1):
- Ask for allergy symptoms - patients who reported allergy symptoms from e.g. grass pollen, tree, weed pollens etc. before administration of Grazax were to be re-scheduled for another visit 2
- Administration of antihistamine (just before intake of Grazax)
- Administration of Grazax with a subsequent 60 minutes observation period.
- Assessment of AEs
- Rating of AEs on Visual Analogue Scale

### Assessments

Safety assessments included recording of all AEs. AEs were rated mild, moderate or severe and furthermore the AEs were rated on a VAS scale.

### Investigational Medicinal Product

The investigational products used in the investigation were Grazax (75,000 SQ-T) and Aerius 2.5 mg.

### Grazax Treatment

| Active ingredients: | *Phleum pratense* grass pollen allergen extract |
|---|---|
| Dosage form: | Oral lyophilisate |
| Dose/strength: | 75,000 SQ-T |

### Antihistamine Treatment

| Active ingredients: | Aerius melting tablet (Desloratadine) |
|---|---|
| Dosage form: | Tablet (disintegrating) |
| Dose/strength: | 2,5 mg |

### Results

A total of 15 patients were included to the screening visit (visit 1). Of these 15 subjects, 9 did not report treatment related adverse events and were therefore not eligible for visit 2. 6 subjects reported local adverse events and consequently they were allowed to attend visit 2.

The overall results are as follows:
All 6 patients reported as mentioned above local treatment related adverse events after Grazax intake on Visit 1. 4 of the patients did not report treatment related adverse events when Grazax was taken in combination with antihistamine (Aerius). One of the 6 patients reported the same AEs on visit 2 and 1 reported that the AE worsened (however, this was very little change on the VAS scale and according to investigator the reaction was unchanged).

The treatment related adverse events are described in the panel below (all AEs are treatment related):

| Patient | AE* Visit 1 | AE Severity | AE* Visit 2 | Outcome |
|---|---|---|---|---|
| 001 | Oral and pharyngeal itching | Mild | No AEs reported | Recovered |
| 002 | Oral itching and tongue swelling | Mild | No AEs reported | Recovered |
| 003 | Pharyngeal itching And Rhinitis | Moderate | No AEs reported | Recovered |
| 004 | Burning mouth and swallowing problems | Mild | Burning mouth and swallowing problems | Recovered |
| 005 | Swelling mouth and tongue and itching mouth | Mild | No AEs reported | Recovered |
| 006 | Itching mouth and pharynx | Mild | Itching mouth and pharynx | Recovered |

| | | | | |
|---|---|---|---|---|
| * (Investigator description) | | | | |

All adverse events reported at visit 1 resolved within minutes to hours. For patient 001, 005, 003 and 002 the AEs resolved within approximately 6 hours, 8 hours, 2 hours and 1 hour respectively. For patient 004 and 006 the AEs lasted below 1 hour.

The present study show that Grazax given in combination with antihistamines (here in the form of an orally disintegrating tablet) reduces the number of treatment related adverse events and that half the amount of the daily dose of desloratidine in a person above 12 years of age (i.e. 2.5 mg) was sufficient to relieve most patients from the itching of the mouth and pharynx.

## Claims

1. A pharmaceutical product comprising one or a plurality of dosage unit(s) where said dosage unit(s) comprises
(a) an allergen,
(b) an antihistamine and
(c) one or more pharmaceutically acceptable excipient(s)
**characterized in that**
one dosage unit comprises one dose of allergen and 0.1 - 5 mg cetirizine, more preferred 0.5 - 5 mg cetirizine, more preferred 1 - 5 mg cetirizine, more preferred 2 - 5 mg cetirizine, more preferred 2.5 - 5 mg cetirizine, more preferred 3 - 5 mg cetirizine or 4 - 5 mg cetirizine, or
one dosage unit comprises one dose of allergen and 0.05 - 2.5 mg desloratidine, more preferred 0.25 - 2.5 mg desloratidine, more preferred 0.5 - 2.5 mg desloratidine, more preferred 1 - 2.5 mg desloratidine, more preferred 1.5 - 2.5 mg desloratodine or 2 - 2.5 mg desloratidine, or
one dosage unit comprises one dose of allergen and 1 - 60 mg fexofenadine, more preferred 2 - 60 mg fexofenadine, more preferred 10 - 60 mg fexofenadine, more preferred 20 - 60 mg fexofenadine, more preferred 30 - 60 mg fexofenadine, more preferred 40 - 60 mg fexofenadine or 50 - 60 mg fexofenadine, or
one dosage unit comprises one dose of allergen and 0.05 - 2.5 mg levocitirizine, more preferred 0.25 - 2.5 mg levozitirizine, more preferred 0.5 - 2.5 mg levozitirizine, more preferred 1- 2.5 mg levozitirizine, more preferred 1.5 - 2.5 mg levozitirizine, or 2 - 2.5 mg levozitirizine, or
one dosage unit comprises one dose of allergen and 0.1 - 5 mg mizolastine, more preferred 0.5 - 5 mg mizolastine, more preferred 1 - 5 mg mizolastine, more preferred 2 - 5 mg mizolastine, more preferred 2.5 - 5 mg mizolastine, more preferred 3 - 5 mg mizolastine,and more preferred 4 - 5 mg mizolastine;
for use in the treatment of allergy in a mammal by sublingual administration.

2. The pharmaceutical product for use according to claim 1, wherein one dosage unit comprises one dose of allergen and 0.1 - 5 mg cetirizine, more preferred 0.5 - 5 mg cetirizine, more preferred 1 - 5 mg cetirizine, more preferred 2 - 5 mg cetirizine, more preferred 2.5 - 5 mg cetirizine, more preferred 3 - 5 mg cetirizine or 4 - 5 mg cetirizine.

3. The pharmaceutical product for use according to claim 1, wherein one dosage unit comprises one dose of allergen and 0.05 - 2.5 mg desloratidine, more preferred 0.25 - 2.5 mg desloratidine, more preferred 0.5 - 2.5 mg desloratidine, more preferred 1 - 2.5 mg desloratidine, more preferred 1.5 - 2.5 mg desloratodine or 2 - 2.5 mg desloratidine.

4. The pharmaceutical product for use according to claim 1, wherein one dosage unit comprises one dose of allergen and 1 - 60 mg fexofenadine, more preferred 2 - 60 mg fexofenadine, more preferred 10 - 60 mg fexofenadine, more preferred 20 - 60 mg fexofenadine, more preferred 30 - 60 mg fexofenadine, more preferred 40 - 60 mg fexofenadine or 50 - 60 mg fexofenadine.

5. The pharmaceutical product for use according to claim 1, wherein one dosage unit comprises one dose of allergen and 0.05 - 2.5 mg levocitirizine, more preferred 0.25 - 2.5 mg levozitirizine, more preferred 0.5 - 2.5 mg levozitirizine, more preferred 1- 2.5 mg levozitirizine, more preferred 1.5 - 2.5 mg levozitirizine, or 2 - 2.5 mg levozitirizine.

6. The pharmaceutical product for use according to claim 1, wherein one dosage unit comprises one dose of allergen and 0.1 - 5 mg mizolastine, more preferred 0.5 - 5 mg mizolastine, more preferred 1 - 5 mg mizolastine, more preferred 2 - 5 mg mizolastine, more preferred 2.5 - 5 mg mizolastine, more preferred 3 - 5 mg mizolastine, and more preferred 4 - 5 mg mizolastine.

7. The pharmaceutical product for use according to any of claims 1-6, wherein the dosage unit(s) are solid dosage unit(s), preferably fast dissolving solid dosage units.

8. The pharmaceutical product for use according to any of claims 1-7 wherein a dosage unit comprises a liquid formulation,

9. The pharmaceutical product for use according to any one of claims 1-8, wherein the allergen is selected from the group consisting of tree pollen allergens, grass pollen allergens, ragweed pollen allergens, mite allergens, insect allergens, venom allergens, animal hair allergens, animal dander allergens and food allergens.

10. The pharmaceutical produc for use according to any of the preceding claims wherein said one dose of allergen is the daily dose of allergen.

11. The pharmaceutical Product for use according to any of claims 1-10 wherein said dosage unit is a lozenge, a tablet, a capsule and a caplet, preferably a fast dissolving tablet.

12. The pharmaceutical product for use according to claims 11 wherein in the dosage unit is a non-compressed tablet.

13. The pharmaceutical product for use according to any one of claims 1-12, where the dosage unit is a solid fast dissolving dosage unit which disintegrates in human saliva within 60 seconds, more preferred within 30 seconds, more preferred within 10 seconds, and most preferred within 2 seconds.

14. The use of (a) an allergen, (b) an antihistamine and (c) pharmaceutically acceptable excipient(s) for the preparation of a pharmaceutical product according to claims 1-13 for the treatment of allergy in a mammal by sublingual administration.

15. The use according to claims 14 wherein the pharmaceutical product comprises a plurality of dosage units and the number of dosage units is the number required for administration in a period of up to 30 days, more preferred in up to 20 days and most preferred in up to 10 days.

16. The use according to claims 14-15 wherein one dosage unit comprises the daily dose of allergen.

17. A dosage unit for use according to any of claims 1-13.

## Patentansprüche

1. Pharmazeutisches Produkt, umfassend eine oder eine Vielzahl von Dosierungseinheit(en), wobei die Dosierungseinheit(en) umfasst,
(a) ein Allergen,
(b) ein Antihistaminikum und
(c) ein oder mehrere pharmazeutisch verträgliche Hilfsstoff(e)
**dadurch gekennzeichnet, dass**
eine Dosierungseinheit eine Dosis des Allergens und 0,1 - 5 mg Cetirizin, mehr bevorzugt 0,5 - 5 mg Cetirizin, mehr bevorzugt 1 - 5 mg Cetirizin, mehr bevorzugt 2 - 5 mg Cetirizin, mehr bevorzugt 2,5 - 5 mg Cetirizin, mehr bevorzugt 3 - 5 mg Cetirizin oder 4 - 5 mg Cetirizin umfasst, oder
eine Dosierungseinheit eine Dosis des Allergens und 0,05 - 2,5 mg Desloratadin, mehr bevorzugt 0,25 - 2,5 mg Desloratadin, mehr bevorzugt 0,5 - 2,5 mg Desloratadin, mehr bevorzugt 1 - 2,5 mg Desloratadin, mehr bevorzugt 1,5 bis 2,5 mg Desloratadin oder 2 - 2,5 mg Desloratadin umfasst, oder
eine Dosierungseinheit eine Dosis des Allergens und 1 - 60 mg Fexofenadin, mehr bevorzugt 2 - 60 mg Fexofenadin, mehr bevorzugt 10 - 60 mg Fexofenadin, mehr bevorzugt 20 - 60 mg Fexofenadin, mehr bevorzugt 30 - 60 mg Fexofenadin, mehr bevorzugt 40 - 60 mg Fexofenadin oder 50 - 60 mg Fexofenadin umfasst, oder
eine Dosierungseinheit eine Dosis des Allergens und 0,05 - 2,5 mg Levocetirizin, mehr bevorzugt 0,25 - 2,5 mg Levocetirizin, mehr bevorzugt 0,5 - 2,5 mg Levocetirizin, mehr bevorzugt 1 - 2,5 mg Levocetirizin, mehr bevorzugt 1,5 bis 2,5 mg Levocetirizin oder 2 - 2,5 mg Levocetirizin umfasst, oder
eine Dosierungseinheit eine Dosis des Allergens und 0,1 - 5 mg Mizolastin, mehr bevorzugt 0,5 - 5 mg Mizolastin, mehr bevorzugt 1 - 5 mg Mizolastin, mehr bevorzugt 2 - 5 mg Mizolastin, mehr bevorzugt 2,5 - 5 mg Mizolastin, mehr bevorzugt 3 - 5 mg Mizolastin, und mehr bevorzugt 4 - 5 mg Mizolastin umfasst;
zur Verwendung in der Behandlung von Allergie bei einem Säugetier durch sublinguale Verabreichung.

2. Pharmazeutisches Produkt zur Verwendung nach Anspruch 1, wobei eine Dosierungseinheit eine Dosis des Allergens und 0,1 - 5 mg Cetirizin, mehr bevorzugt 0,5 - 5 mg Cetirizin, mehr bevorzugt 1 - 5 mg Cetirizin, mehr bevorzugt 2 - 5 mg Cetirizin, mehr bevorzugt 2,5 - 5 mg Cetirizin, mehr bevorzugt 3 - 5 mg Cetirizin oder 4 - 5 mg Cetirizin umfasst.

3. Pharmazeutisches Produkt zur Verwendung nach Anspruch 1, wobei eine Dosierungseinheit eine Dosis des Allergens und 0,05 - 2,5 mg Desforatadin, mehr bevorzugt 0,25 - 2,5 mg Desloratadin, mehr bevorzugt 0,5 - 2,5 mg Desloratadin, mehr bevorzugt 1 - 2,5 mg Desloratadin, mehr bevorzugt 1,5 bis 2,5 mg Desloratadin oder 2 - 2,5 mg Desloratadin umfasst.

4. Pharmazeutisches Produkt zur Verwendung nach Anspruch 1, wobei eine Dosierungseinheit eine Dosis des Allergens und 1 - 60 mg Fexofenadin, mehr bevorzugt 2 - 60 mg Fexofenadin, mehr bevorzugt 10 - 60 mg Fexofenadin, mehr bevorzugt 20 - 60 mg Fexofenadin , mehr bevorzugt 30 - 60 mg Fexofenadin, mehr bevorzugt 40 - 60 mg Fexofenadin oder 50 - 60 mg Fexofenadin umfasst.

5. Pharmazeutisches Produkt zur Verwendung nach Anspruch 1, wobei eine Dosierungseinheit eine Dosis des Allergens und 0,05 - 2,5 mg Levocetirizin, mehr bevorzugt 0,25 - 2,5 mg Levocetirizin, mehr bevorzugt 0,5 - 2,5 mg Levocetirizin, mehr bevorzugt 1 - 2,5 mg Levocetirizin, mehr bevorzugt 1,5 bis 2,5 mg Levocetirizin oder 2 - 2,5 mg Levocetirizin umfasst.

6. Pharmazeutisches Produkt zur Verwendung nach Anspruch 1, wobei eine Dosierungseinheit eine Dosis des Allergens und 0,1 - 5 mg Mizolastin, mehr bevorzugt 0,5 - 5 mg Mizolastin, mehr bevorzugt 1 - 5 mg Mizolastin, mehr bevorzugt 2 - 5 mg Mizolastin, mehr bevorzugt 2,5 - 5 mg Mizolastin, mehr bevorzugt 3 - 5 mg Mizolastin, und mehr bevorzugt 4 - 5 mg Mizolastin umfasst.

7. Pharmazeutisches Produkt zur Verwendung nach einem der Ansprüche 1 - 6, wobei die Dosierungseinheit(en) feste Dosierungseinheit(en), vorzugsweise schnell auflösende feste Dosierungseinheiten, sind.

8. Pharmazeutisches Produkt zur Verwendung nach einem der Ansprüche 1 - 7, wobei eine Dosierungseinheit eine flüssige Formulierung umfasst.

9. Pharmazeutisches Produkt zur Verwendung nach einem der Ansprüche 1 - 8, wobei das Allergen ausgewählt ist aus der Gruppe, bestehend aus Baumpollenallergene, Gräserpollenallergene, Ambrosiapollenallergene, Milbenallergene, Insektenallergene, Giftallergene, Tierhaarallergene, tierische Hautschuppenallergene und Nahrungsmittelallergene.

10. Pharmazeutisches Produkt zur Verwendung nach einem der vorhergehenden Ansprüche, wobei die eine Allergen-Dosis die Tagesdosis des Allergens ist.

11. Pharmazeutisches Produkt zur Verwendung nach einem der Ansprüche 1 - 10, wobei die Dosierungseinheit eine Pastille, eine Tablette, eine Kapsel und ein Caplet, vorzugsweise eine schnell auflösende Tablette ist.

12. Pharmazeutisches Produkt zur Verwendung nach Anspruch 11, wobei in der Dosierungseinheit eine nicht-komprimierte Tablette ist.

13. Pharmazeutisches Produkt zur Verwendung nach einem der Ansprüche 1 - 12, wobei die Dosierungseinheit eine feste schnell lösende Dosierungseinheit ist, die im menschlichen Speichel innerhalb von 60 Sekunden zerfällt, mehr bevorzugt innerhalb von 30 Sekunden, mehr bevorzugt innerhalb von 10 Sekunden, und am meisten bevorzugt innerhalb von 2 Sekunden.

14. Verwendung von (a) einem Allergen, (b) einem Antihistaminikum und (c) pharmazeutisch verträglichen Hilfsstoff(en) zur Herstellung eines pharmazeutischen Produkts nach Ansprüchen 1 - 13 zur Behandlung von Allergie bei einem Säugetier durch sublinguale Verabreichung.

15. Verwendung nach Anspruch 14, wobei das pharmazeutische Produkt eine Vielzahl von Dosierungseinheiten umfasst und die Anzahl der Dosierungseinheiten die erforderliche Anzahl für die Verabreichung in einem Zeitraum von bis zu 30 Tagen, mehr bevorzugt in bis zu 20 Tagen und am meisten bevorzugt in bis zu 10 Tage ist.

16. Verwendung nach den Ansprüchen 14 -15, wobei eine Dosierungseinheit die tägliche Dosis an Allergen umfasst.

17. Dosierungseinheit zur Verwendung nach einem der Ansprüche 1 - 13.

## Revendications

1. Produit pharmaceutique comprenant une ou une pluralité d'unités de dosage, où ladite ou lesdites unités de dosage comprennent
(a) un allergène,
(b) un antihistaminique, et
(c) un ou plusieurs excipients pharmaceutiquement acceptables,
**caractérisé en ce que**
une unité de dosage comprend une dose d'allergène et 0,1-5 mg de cétirizine, plus préférablement 0,5-5 mg de cétirizine, plus préférablement 1-5 mg de cétirizine, plus préférablement 2-5 mg de cétirizine, plus préférablement 2,5-5 mg, de cétirizine, plus préférablement 3-5 mg de cétirizine ou 4-5 mg de cétirizine, ou
une unité de dosage comprend une dose d'allergène et 0,05-2,5 mg de desloratidine, plus préférablement 0,25-2,5 mg de desloratidine, plus préférablement 0,5-2,5 mg de desloratidine, plus préférablement 1,5-2,5 mg de desloratidine, plus préférablement 1,5-2,5 mg de desloratidine ou 2-2,5 mg de desloratidine, ou
une unité de dosage comprend une dose d'allergène et 1-60 mg de féxofénadine, plus préférablement 2-60 mg de féxofénadine, plus préférablement 10-60 mg de féxofénadine, plus préférablement 20-60 mg de féxofénadine, plus préférablement 30-60 mg de féxofénadine, plus préférablement 40-60 mg de féxofénadine ou 50-60 mg de féxofénadine, ou
une unité de dosage comprend une dose d'allergéne et 0,05-2,5 mg de lévocétirizine, plus préférablement 0,25-2,5 mg de lévocétirizine, plus préférablement 0,5-2,5 mg de lévocétirizine, plus préférablement 1-2,5 mg de lévocétirizine, plus préférablement 1,5-2,5 mg de lévocétirizine ou 2-2,5 mg de lévocétirizine, ou
une unité de dosage comprend une dose d'allergène et 0,1-5 mg de mizolastine, plus préférablement 0,5-5 mg de mizolastine, plus préférablement 1-5 mg de mizolastine, plus préférablement 2-5 mg de mizolastine, plus préférablement 2,5-5 mg de mizolastine, plus préférablement 3-5 mg de mizolastine ou plus préférablement 4-5 mg de mizolastine,
pour une utilisation dans le traitement d'une allergie chez un mammifère par administration sublinguale.

2. Produit pharmaceutique pour une utilisation selon la revendication 1, dans lequel une unité de dosage comprend une dose d'allergène et 0,1-5 mg de cétirizine, plus préférablement 0,5-5 mg de cétirizine, plus préférablement 1-5 mg de cétirizine, plus préférablement 2-5 mg de cétirizine, plus préférablement 2,5-5 mg de cétirizine, plus préférablement 3-5 mg de cétirizine ou 4-5 mg de cétirizine.

3. Produit pharmaceutique pour une utilisation selon la revendication 1, dans lequel une unité de dosage comprend une dose d'allergène et 0,05-2,5 mg de desloratidine, plus préférablement 0,25-2,5 mg de desloratidine, plus préférablement 0,5-2,5 mg de desloratidine, plus préférablement 1-2,5 mg de desloratidine, plus préférablement 1,5-2,5 mg de desloratidine ou 2-2,5 mg de desloratidine.

4. Produit pharmaceutique pour une utilisation selon la revendication 1, dans lequel une unité de dosage comprend une dose d'allergéne et 1-60 mg de féxofénadine, plus préférablement 2-60 mg de féxofénadine, plus préférablement 10-60 mg de féxofénadine, plus préférablement 20-60 mg de féxofénadine, plus préférablement 30-60 mg de féxofénadine, plus préférablement 40-60 mg de féxofénadine ou 50-60 mg de féxofénadine.

5. Produit pharmaceutique pour une utilisation selon la revendication 1, dans lequel une unité de dosage comprend une dose d'allergène et 0,05-2,5 mg de lévocétirizine, plus préférablement 0,25-2,5 mg de lévocétirizine, plus préférablement 0,5-2,5 mg de lévocétirizine, plus préférablement 1-2,5 mg de lévocétirizine, plus préférablement 1,5-2,5 mg de lévocétirizine ou 2-2,5 mg de lévocétirizine.

6. Produit pharmaceutique pour une utilisation selon la revendication 1, dans lequel une unité de dosage comprend une dose d'allergène et 0,1-5 mg de mizolastine, plus préférablement 0,5-5 mg de mizolastine, plus préférablement 1-5 mg de mizolastine, plus préférablement 2-5 mg de mizolastine, plus préférablement 2,5-5 mg de mizolastine, plus préférablement 3-5 mg de mizolastine ou plus préférablement 4-5 mg de mizolastine.

7. Produit pharmaceutique pour une utilisation selon l'une quelconque des revendications 1-6, dans lequel la ou les unités de dosage sont des unités de dosage solides, préférablement des unités de dosage solides à dissolution rapide.

8. Produit pharmaceutique pour une utilisation selon l'une quelconque des revendications 1-7, dans lequel une unité de dosage comprend une formulation liquide.

9. Produit pharmaceutique pour une utilisation selon l'une quelconque des revendications 1-8, dans lequel l'allergène est choisi dans le groupe constitué par les allergènes du pollen des arbres, les allergènes du pollen des graminées, les allergènes du pollen de l'ambroisie, les allergènes des acariens, les allergènes des insectes, les allergènes des venins, les allergènes des poils d'animaux, les allergènes des produits de desquamation animaux, et les allergènes alimentaires.

10. Produit pharmaceutique pour une utilisation selon l'une quelconque des revendications précédentes, dans lequel ladite dose d'allergène est la dose journalière d'allergène.

11. Produit pharmaceutique pour une utilisation selon l'une quelconque des revendications 1-10, dans lequel ladite unité de dosage est une pastille, un comprimé, une capsule et un caplet, préférablement un comprimé a dissolution rapide.

12. Produit pharmaceutique pour une utilisation selon la revendication 11, dans lequel, dans l'unité de dosage, on trouve un comprimé non compressé.

13. Produit pharmaceutique pour une utilisation selon l'une quelconque des revendications 1-12, dans lequel l'unité de dosage est une unité de dosage solide à dissolution rapide, qui se désintègre dans la salive humaine en 60 secondes, plus préférablement en 30 secondes, plus préférablement en 10 secondes, et tout préférablement en 2 secondes.

14. Utilisation (a) d'un allergène, (b) d'un antihistaminique et (c) d'un ou plusieurs excipients pharmaceutiquement acceptables, pour la préparation d'un produit pharmaceutique selon les revendications 1-13 pour le traitement d'une allergie chez un mammifère par administration sublinguale.

15. Utilisation selon la revendication 14, dans laquelle le produit pharmaceutique comprend une pluralité dignités de dosage et le nombre d'unités de dosage est le nombre requis pour une administration sur une période allant jusqu'à 30 jours, plus préférablement allant jusqu'à 20 jours et tout préférablement jusqu'à 10 jours.

16. Utilisation selon les revendications 14-15, dans laquelle une unité de dosage comprend la dose journalière d'allergène.

17. Unité de dosage pour une utilisation selon l'une quelconque revendications 1-13.
